(19) Europäisches Patentamt
European Patent Office
Office européen des brevets

(11) **EP 2 208 457 B1**

(12) **EUROPÄISCHE PATENTSCHRIFT**

(45) Veröffentlichungstag und Bekanntmachung des Hinweises auf die Patenterteilung:
**27.02.2013 Patentblatt 2013/09**

(21) Anmeldenummer: **09015466.7**

(22) Anmeldetag: **15.12.2009**

(51) Int Cl.:
**A61B 3/09** *(2006.01)*

(54) **Verfahren und Vorrichtung zur Bestimmung der individuell erforderlichen Addition einer Sehhilfe**

Method and device for determining the individual necessary addition of a visual aid

Procédé et dispositif de détermination de l'ajout individuel nécessaire d'une aide à la vision

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **16.01.2009 DE 102009004866**

(43) Veröffentlichungstag der Anmeldung:
**21.07.2010 Patentblatt 2010/29**

(73) Patentinhaber: **Carl Zeiss Vision GmbH 73430 Aalen (DE)**

(72) Erfinder:
• **Welscher, Monique, B.**
**73433 Aalen (DE)**
• **Cabeza-Guillèn, Jesus-Miguel**
**73434 Aalen (DE)**
• **Kratzer, Timo**
**73434 Aalen (DE)**

(74) Vertreter: **Braunger, Dieter Carl Zeiss AG Patentabteilung Carl-Zeiss-Straße 22 73447 Oberkochen (DE)**

(56) Entgegenhaltungen:
• **MILLODOT M ET AL: "Presbyopia correction and the accommodation in reserve" OPHTHALMIC & PHYSIOLOGICAL OPTICS UK, Bd. 9, Nr. 2, April 1989 (1989-04), Seiten 126-132, XP002574603 ISSN: 0275-5408**
• **WOLD JON E ET AL: "Subjective and objective measurement of human accommodative amplitude." JOURNAL OF CATARACT AND REFRACTIVE SURGERY OCT 2003, Bd. 29, Nr. 10, Oktober 2003 (2003-10), Seiten 1878-1888, XP002574604 ISSN: 0886-3350**
• **LEFFLER CHRISTOPHER T ET AL: "Clinical predictors of the optimal spectacle correction for comfort performing desktop tasks." CLINICAL & EXPERIMENTAL OPTOMETRY : JOURNAL OF THE AUSTRALIAN OPTOMETRICAL ASSOCIATION NOV 2008, Bd. 91, Nr. 6, November 2008 (2008-11), Seiten 530-537, XP002574605 ISSN: 0816-4622**
• **WIN-HALL D M ET AL: "Objective accommodation measurements in prepresbyopic eyes using an autorefractor and an aberrometer" JOURNAL CATARACT AND REFRACTIVE SURGERY, SURGERY, FAIRFAX, VA, Bd. 34, Nr. 5, 1. Mai 2008 (2008-05-01), Seiten 774-784, XP022651183 ISSN: 0886-3350 [gefunden am 2008-04-28]**
• **ANTONA BEATRIZ ET AL: "Comparing methods of determining addition in presbyopes." CLINICAL & EXPERIMENTAL OPTOMETRY : JOURNAL OF THE AUSTRALIAN OPTOMETRICAL ASSOCIATION MAY 2008, Bd. 91, Nr. 3, Mai 2008 (2008-05), Seiten 313-318, XP002574606 ISSN: 0816-4622**

**Beschreibung**

Die Erfindung betrifft ein Verfahren zur Bestimmung der individuell erforderlichen Addition einer Sehhilfe, eine Vorrichtung zur Bestimmung der individuell erforderlichen Addition einer Sehhilfe, und ein Computerprogramm mit Programmcode eingerichtet zur Ausführung des Verfahrens.

Nachfolgend wird zunächst eine Mehrzahl an Begriffen definiert, auf welche im Weiteren Bezug genommen wird:

Die Sehschärfe oder Visus V ist das Ausmaß der Fähigkeit eines Lebewesens, mit seinem Sehorgan Muster und Konturen in der Außenwelt als solche wahrzunehmen. Die dimensionslose Eigenschaft Visus V wird definiert zu

$$V = 1' / \text{(individuelle angulare Sehschärfe)}, \tag{1}$$

wobei die Winkel-Sehschärfe das Auflösungsvermögen ist, bei dem zwei Sehobjekte noch als getrennt wahrgenommen werden (sog. Minimum Separabile).

Die Schärfentiefe oder Abbildungstiefe T des Auges ist nach DIN 19040, 5-3.12 die Größe des Bereiches vor und hinter dem Einstellpunkt $A_E$, in dem die Objektpunkte mit einer nicht wahrnehmbaren Unschärfe des Netzhautbildes abgebildet werden. Die Abbildungstiefe T des Auges hängt vom Pupillendurchmesser und von der zentralen Sehschärfe ab. Bei einem Pupillendurchmesser von 2,9 mm und einer Sehschärfe V von 1,0 ergibt sie sich theoretisch aus den 0,1 dpt entsprechenden Strecken beiderseits des Einstellpunktes $A_E$: 10 cm in Lichtrichtung "hinter" dem Einstellpunkt, 10 cm in Lichtrichtung "vor" dem Einstellpunkt ergibt eine Abbildungstiefe von 20 cm, das entspricht ca. 0,25 dpt (Quelle: Helmut Goersch, Wörterbuch der Optometrie, 2. Auflage 2001).

Gebrauchsentfernung $a_{Gebrauch}$ ist die von einem Probanden gewählte Entfernung vom Augenhauptpunkt $H_A$ zum Objekt.

Unter Addition Add oder Nahzusatz versteht man die Differenz zwischen der sphärischen Wirkung des Nahteils und der des Fernteils eines Gleitsicht- oder Mehrstärkenbrillenglases.

Akkommodation (von lateinisch accomodare "anpassen, adaptieren, anlegen, festmachen") bezeichnet Vorgänge zur Änderung des Lichtwegs innerhalb des Auges, um Gegenstände unterschiedlicher Entfernung in der Netzhautebene scharf abzubilden. Bei Säugetieren und Vögeln wird nach einer Theorie von Helmholtz zur Akkommodation die Form der elastischen Linse verändert, um die Brechkraft zu variieren. Eine Theorie von Schachar geht zusätzlich von einer Vorverlagerung der Augenlinse bei der Akkommodation aus.

Gleichzeitig mit der Anspannung des Ziliarmuskels vollziehen die Augen eine Konvergenzbewegung, das heißt, die Augen bewegen sich zueinander hin, so dass sich die Sehachsen im Fixierpunkt schneiden. Diese Konvergenzbewegung ist u. a. Voraussetzung für die Fusion der Seheindrücke beider Augen in der Nähe. Weiterhin stellt sich eine Pupillenverengung (Konvergenzmiosis) ein. Die drei gekoppelten Reaktionen bei Einstellung der Augen auf ein nahes Objekt: Akkommodation, Konvergenz und synergischer Pupillenreflex bezeichnet man als Akkommodationstrias oder Konvergenzreaktion.

Die Fernpunktrefraktion $A_R$ ist der Kehrwert des Fernpunktabstands $a_R$, also der Entfernung $a_{Objekt}$ des Orts des Objekts vom objektseitigen Hauptpunkt $H_A$ des Auges, in dem das akkommodationslose Auge das Objekt scharf wahrnimmt. Die Einstellpunktrefraktion $A_E$ ist der Kehrwert des Einstellpunktabstands $a_E$ (veraltet: Akkommodationsentfernung), also des Abstands $a_{Objekt}$ des Objektpunkts, der im momentanen Akkommodationszustand in der Mitte der Netzhautgrube abgebildet und damit scharf gesehen wird.

Als Akkommodationsgeschwindigkeit $v_A$ in dpt/s bezeichnet man die akkommodationsbedingte Änderung der Einstellpunktrefraktion $A_E$ pro Zeiteinheit. Die Akkommodationsgeschwindigkeit $v_A$ ergibt sich aus dem Verhältnis von Änderung der Einstellpunktrefraktion $A_E$ zur Akkommodationszeit $t_A$. Sie hängt vom Lebensalter und von den Einstellpunktrefraktionen $A_{Ev}$, $A_{En}$ vor und nach der Akkommodation ab und beträgt ungefähr 2 bis 5 dpt/s.

Unter dem in Dioptrie (dpt) angegebenen Akkommodationsbedarf A versteht man nach der DIN 5340-15 den Kehrwert des in Metern angegebenen Objektabstandes $a_{Objekt}$ vom objektseitigen Hauptpunkt $H_A$ des Auges. Befindet sich vor dem Auge ein optisches System, SQ..tritt.. an die Stelle des Objektabstandes $a_{Objekt}$ der Abstand des von diesem System entworfenen Bildes.

Die relative Akkommodation $\Delta A_{rel}$ ist nach der DIN 5340-12 definiert als die durch Gläser mit sphärischer Wirkung erzwungene Änderung des Akkommodationserfolges $\Delta A$ bei unveränderter Vergenzstellung und unverändert scharfem binokularen Einfachsehen.

Unter psychischer Akkommodation $\Delta A_{psych}$ (proximale Akkommodation = Instrumentenmyopie) versteht man die durch nahe reale Objekte aufgrund des Bewusstseins der Nähe ausgelöste Nahakkommodation $A_{Nah}$

Der Akkommodationsreiz $\Delta A_{Reiz}$ ist der bei einer Änderung der Fixationsentfernung $a_{Objekt}$ durch das dann zuerst unscharfe Netzhautbild ausgelöste Reiz zur Akkommodation.

Unter dem ebenfalls in Dioptrie (dpt) angegebenen Akkommodationserfolg $\Delta A$ (veraltet: äußere Akkommoda-

tion) versteht man nach der DIN 5340-99 die Differenz zwischen Fernpunktrefraktion $A_R$ und Einstellpunktrefraktion $A_E$.

Die DIN 5340-20 definiert den maximalen Akkommodationserfolg oder die Akkommodationsbreite $\Delta A_{max}$ als Differenz zwischen Fernpunktrefraktion $A_R$ und Nahpunktrefraktion $A_p$. Die Nahpunktrefraktion $A_p$ ist der Kehrwert des Abstands $a_p$ des Augenhauptpunkts $H_A$ zum scharf gesehenen Objektpunkt bei stärkster Akkommodation.

Bei Kleinkindern beträgt die Akkommodationsbreite $\Delta A_{max}$ ca. 14 dpt. Bezogen auf die Gesamtbrechkraft des Auges von ca. 58 dpt entspricht dies einer Variation von ca. 25 %. Im hohen Alter fällt die Akkommodationsbreite $\Delta A_{max}$ auf Werte unter 2 dpt bzw. 4 % ab. Dadurch vergrößert sich der kleinste Abstand, der Nahpunkt $a_P$, in dem Gegenstände noch scharf gesehen werden können, von ca. $a_P = 7$ cm bei Kleinkindern auf mehr als $a_P = 50$ cm im hohen Alter.

Eine exakte Trennung zwischen Akkommodationsbreite $\Delta A_{max}$ und Schärfentiefe T des menschlichen Auges ist bis dato nicht möglich. Die Summe aus tatsächlicher, durch die o.a. Mechanismen des Akkommodationstrias definierter Akkommodationsbreite $\Delta A_{max}$ und Schärfentiefe T wird daher nachfolgend als physiologische Akkommodationsbreite $\Delta A_{max}$ bezeichnet.

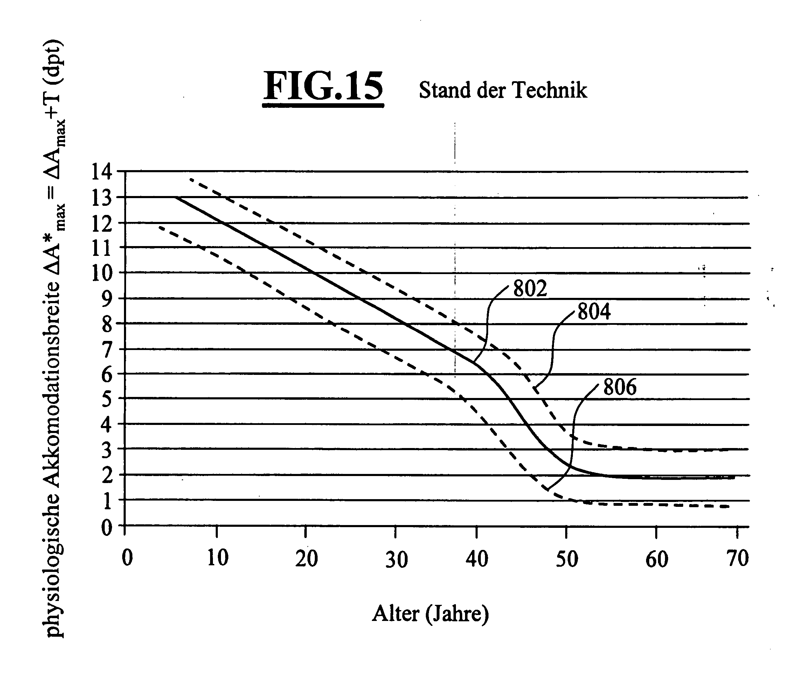

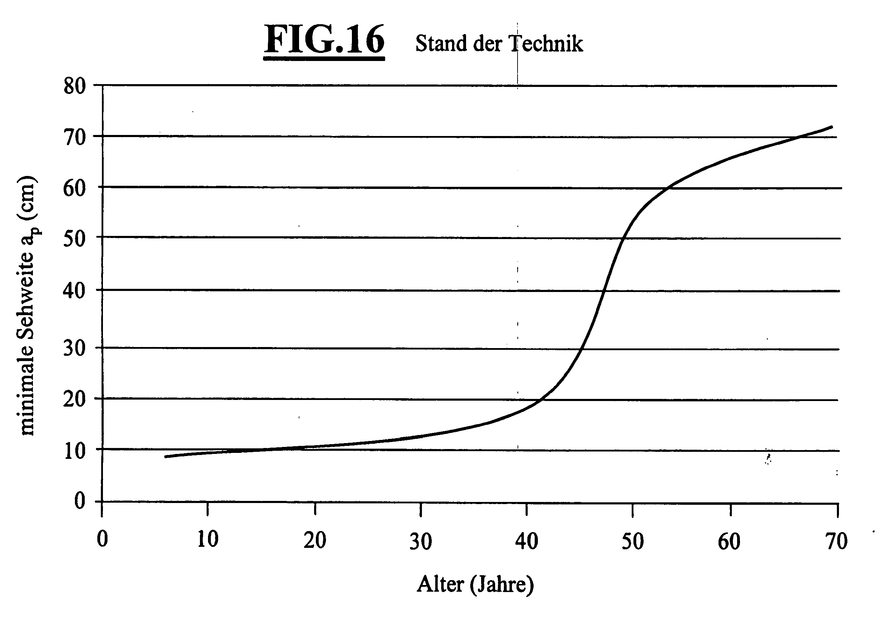

Im Jahr 1922 hat Duane anhand der Seheindrücke von 5000 normalsichtigen Probanden die Altersabhängigkeit der durchschnittlichen physiologischen Akkommodationsbreite $\Delta A^*_{max, m}$ ermittelt. Bei der Ermittlung dieser Daten wurde also nicht zwischen Akkommodationsbreite $\Delta A_{max}$ und Schärfentiefe T unterschieden, d.h. die ermittelte Kurve ist eine Überlagerung beider Effekte. Die mittlere Kurve 802 des Diagramms nach der Figur 15 zeigt diese Altersabhängigkeit der durchschnittlichen physiologischen Akkommodationsbreite $\Delta A^*_{max, m}$. Die obere und die untere Kurve 804, 806 repräsentieren jeweils die physiologische Grenze der Dispersion. Das Diagramm nach der Figur 16 zeigt die entsprechende Altersabhängigkeit der minimalen Sehweite $a_p$.

Ursächlich für die Abnahme der Akkommodationsbreite $\Delta A_{max}$ ist eine im zunehmenden Alter herabgesetzte Elastizität der Linsenkapsel bzw. eine Linsenverdickung durch lebenslanges Wachstum der Linsenschale (Helmholtz-Theorie). Es wurde festgestellt, dass selbst bei verschwindender Elastizität der Linsenkapsel ein Rest an Akkommodationsbreite $\Delta A_{max}$ verbleibt. Die sogenannte Schachar-Theorie, nach der zusätzlich von einer Vorverlagerung der Augenlinse bei der Akkommodation ausgegangen wird, kann den verbleibenden Rest an Akkommodationsbreite $\Delta A_{max}$ erklären, welcher auch mit zunehmendem Alter nicht verloren geht.

Fällt die Akkommodationsbreite $\Delta A_{max}$ inkl. Schärfentiefe T mit zunehmendem Alter (siehe Figur 15) unter etwa 3 Dioptrien (die Zeitung muss zum Lesen mit Fernbrille in über 35 cm Abstand gehalten werden) spricht man von einer Alterssichtigkeit (Presbyopie). Eine einfache Lesebrille, eine Bifokalbrille, eine Gleitsichtbrille oder Mehrstärken-Kontaktoder Intraokularlinsen können die Presbyopie ausgleichen.

Unter Gebrauchsakkommodation $\Delta A_{Gebrauch}$ versteht man diejenige Akkommodation A, welche über einen längeren Zeitraum ohne Beschwerden aufgebracht werden kann. Sie beträgt etwa ½ (Reiner) bis 2/3 (Schober) der Akkommodationsbreite $\Delta A_{max}$.

Aus dem Stand der Technik ist eine Vielzahl an Verfahren zur Ermittlung einer für eine fehlsichtige Person geeignet und an deren Bedürfnissen angepassten Addition $A_{dd}$ einer multifokalen oder progressiven Brillen-, Kontaktoder Intraokularlinse bekannt. Alle diese Verfahren haben gemein, dass die Addition Add den Kehrwert der minimalen Gebrauchsentfernung $a_{Gebrauch,min}$ nicht überschreiten sollte. Sie unterscheiden sich jedoch in der Bestimmung des tatsächlichen Werts der Addition Add. Dieser Sachverhalt wird nachfolgend anhand eines einfachen Rechenbeispiels erläutert.

Es wird angenommen, ein Proband empfindet es als angenehm, beim Lesen ein Schriftstück in einem nachfolgend als Gebrauchsabstand $a_{Gebrauch}$ bezeichneten Abstand von 40 cm zum Auge zu halten. Weiterhin wird angenommen, dass der minimale Gebrauchsabstand $a_{Gebrauch,mine}$ in dem der Proband das Schriftstück jemals hält, 33 cm beträgt. Der Kehrwert des minimalen Gebrauchsabstands $a_{Gebrauch,min}$, beträgt 3 dpt. Diese 3 dpt stellen den Akkommodationsbedarf A für die minimale Gebrauchsentfernung $a_{Gebrauch,min}$ dar.

Wäre der Proband z.B. 50 Jahre alt, so besäße er nach dem Diagramm von Duane nach der Figur 15 eine Restakkommodationsbreite $\Delta A_{max}$ von etwa 2 dpt. Weil der tatsächlich verwendete Akkommodationsaufwand, die Gebrauchsakkommodation $\Delta A_{Gebrauch}$, nach der Theorie von Reiner etwa die Hälfte (nach der Theorie von Schober etwa zwei Drittel) der noch vorhandenen Akkommodationsbreite $\Delta A_{max}$ beträgt, würde er etwa 1 dpt (bis 1,5 dpt nach Schober) seiner Akkommodationsbreite $\Delta A_{max}$ tatsächlich auch verwenden. Die richtige Addition Add für eine Brillen-, Kontakt oder Intraokularlinse für den Probanden bei dem vorstehend angegebenen minimalen Gebrauchsabstand $a_{Gebrauch, min}$ des Probanden von 33 cm würde also 2 dpt nach Reiner (bzw. 1,5 dpt nach Schober) betragen.

Weil sich in diesem Beispiel die Schriftstücke beim Lesen jedoch überwiegend in einer Gebrauchsentfernung $a_{Gebrauch}$ (vorliegend der Leseentfernung) von 40 cm zum Auge des Probanden (der Kehrwert $1/a_{Gebrauch}$ hiervon ist 2,5 dpt) befinden, würde bei einer Gebrauchsakkommodation $\Delta A_{Gebrauch}$ von etwa 1 dpt (bzw. 1,5 dpt nach Schober) eine Addition Add von 1,5 dpt (bzw. 1 dpt nach Schober) in zufriedenstellender Weise die Bedürfnisse des Probanden befriedigen. Dank seiner Restakkommodationsbreite $\Delta A_{max}$ (einschließlich Schärfentiefe T) ist er in der Lage in einer Leseentfernung $a_{Gebrauch}$ von 33 cm mit einer Addition Add von lediglich 1,5 dpt (bzw. 1 dpt. nach Schober) scharf zu sehen. Die an die Bedürfnisse des Probanden am Besten angepasste Addition Add der Brillen-, Kontakt oder Intraokularlinse beträgt also nach der Theorie von Reiner 1,5 dpt (bzw. 1,0 dpt nach Schober) und sollte 2,0 dpt nicht übersteigen.

Bedauerlicherweise werden in der Praxis heutzutage zumeist höhere Additionen Add verschrieben, was häufig zu einer Unzufriedenheit des Trägers der Sehhilfe mit dem resultierenden Linsendesign führt.

Nachfolgende Abschnitte stellen einen Überblick über die nach Auffassung der Erfinder am häufigsten benutzten Verfahren zur Bestimmung der Addition Add einer Brillen-, Kontakt - oder Intraokularlinse und die jeweiligen Unzulänglichkeiten der Methoden dar.

## 1. Additionsbestimmung anhand einer Schätztabelle

Die am häufigsten verwendete Methode zur Additionsbestimmung bedient sich einer nachfolgend als Tabelle 1 wiedergegebenen Schätztabelle.

Tabelle 1: Schätztabelle zur Additionsbestimmung einer Brillen-, Kontakt- oder Intraokularlinse

| Alter (Jahre) | Addition Add (dpt) für eine Gebrauchsentfernung von 33 cm | Addition Add (dpt) für eine Gebrauchsentfernung von 40 cm |
| --- | --- | --- |
| 45 | 1,0 | 0,75 |
| 45 ... 48 | 1,5 | 1,00 |
| 48 ... 50 | 2,0 | 1,25 |
| 50 ... 65 | 2,5 | 1,75 |

Diese Schätztabelle orientiert sich an der mittleren physiologischen Akkommodationsbreite $\Delta A^*_{max,\,m}$ der Altersstufe und der gewünschten Gebrauchsentfernung $a_{Gebrauch}$. Sie basiert auf der oben beschriebenen und in der Figur 15 dargestellten Kurve nach Duane.

Bei diesem Verfahren wird zunächst die für den Probanden geeignete Gebrauchsentfernung $a_{Gebrauch}$ bestimmt. Diese stellt die vom Probanden am häufigsten gebrauchte Entfernung zwischen dem objektseitigen Hauptpunkt $H_A$ des Auges und wahrzunehmendem Objekt dar. Die Addition Add wird dann entsprechend dem Alter des Probanden anhand der oben gezeigten Tabelle abgeschätzt.

Da dieses Verfahren allein auf Schätzungen beruht und nur das Alter und die Gebrauchsentfernung $a_{Gebrauch}$ berücksichtigt, ist es sehr ungenau.

Die Unzulänglichkeit dieser Methode wurde bereits früh erkannt. Es wurden Methoden entwickelt, die eine Überprüfung und gegebenenfalls auch eine Korrektur der anhand der Schätztabelle bestimmten Addition Add ermöglichen. Nachfolgend werden einige ausgewählte Beispiele vorgestellt.

## 2. Additionsbestimmung anhand der Schätztabelle nach 1. mit Rot-Grün-Kontrastausgleich

Bei diesem Verfahren werden dem Probanden in der entsprechenden Gebrauchsentfernung $a_{Gebrauch}$ binokular rote und grüne Optotypen mit der nach dem unter 1. beschriebenen Verfahren ermittelten Addition $Add_{vorläufig}$ entsprechenden dioptrischen Wirkung präsentiert. Der Proband soll die Optotypen auf Kontrastgleichheit vergleichen. Konkret vergleicht der Proband die Helligkeiten der roten und grünen Foki der Optotypen. Wenn der rote Fokus der Optotypen dunkler erscheint, muss die Addition Add verringert oder der Objektabstand $a_{Gebrauch}$ erhöht werden. Wenn der grüne Fokus der Optotypen dunkler erscheint, muss die Addition Add vergrößert oder der Objektabstand $a_{Gebrauch}$ verringert werden. Wenn die Helligkeiten der Optotypen mit roten und grünen Foki gleich empfunden werden, ist die zugehörige Addition Add richtig.

Auch diese Methode ist sehr ungenau, da sie ebenfalls auf der Verwendung der o.a. Schätztabelle beruht. Darüber hinaus beruht sie auf der subjektiven Empfindung des Probanden. Gibt der Proband an, alle Optotypen spontan mit derselben Helligkeit zu sehen, ist der Optiker gehalten, den Objektabstand $a_{Gebrauch}$ zu verändern, um zu prüfen, ob der Proband auf den Test überhaupt reagiert. Darüber hinaus hat sich herausgestellt, dass unter 50-jährige Probanden nicht exakt auf die Objektebene akkommodieren, sondern geringfügig vor diese. Die Differenz wird durch die Schärfentiefe T des menschlichen Auges ausgeglichen mit dem Effekt, dass der Optiker eine zu hohe Addition Add ermittelt. Je nach Alter wird daher häufig ein Korrekturbetrag entsprechend der nachfolgend angegebenen Tabelle 2 abgezogen:

Tabelle 2: Korrekturbetrag für nach Rot-Grün-Kontrastausgleich bestimmte Addition Add einer Brillen-, Kontakt- oder Intraokularlinse

| Alter (Jahre) | Korrekturbetrag (dpt) |
| --- | --- |
| unter 50 | 0,5 |

(fortgesetzt)

| Alter (Jahre) | Korrekturbetrag (dpt) |
|---|---|
| 50 ... 60 | 0,25 |

Da dieses Verfahren allein auf Schätzungen beruht und nur das Alter, die Gebrauchsentfernung $a_{Gebrauch}$ und den binokularen Kontrast berücksichtigt, ist es sehr ungenau.

### 3. Additionsbestimmung unter Berücksichtigung der subjektiv bestimmten Akkommodationsbreite

Bei diesem Verfahren wird dem Probanden eine geeignete Sehprobe (z.B. die sogenannte Duane'sche Strichfigur) in ausreichend großer Entfernung dargeboten. Dann wird die Entfernung zwischen Auge und Sehprobe kontinuierlich verringert, bis der Proband angibt, die Sehprobe unscharf wahrzunehmen. Der Kehrwert dieser Entfernung zwischen Objekt und Hauptpunkt $H_A$ entspricht der Akkommodationsbreite $\Delta A_{max}$ inklusive der Schärfentiefe T des Auges. Als Addition Add nimmt man einem Vorschlag von Reiner folgend die Hälfte dieses Kehrwerts. Reiner geht nämlich davon aus, dass die tatsächliche Gebrauchsakkommodation $\Delta A_{Gebrauch}$ gerade die Hälfte der Akkommodationsbreite $\Delta A^*_{max}$ beträgt. Schober schlägt anstelle des Faktors ½ einen Faktor $^2/_3$ vor.

Wenn der Test monokular durchgeführt wird, wird der Akkommodationsstimulus (Akkommodationsreiz $\Delta A_{Reiz}$) allein durch die retinale Unschärfe und das Bewusstsein der Nähe ausgelöst, wohingegen beim binokularen Test auch die Konvergenz der Augen den Akkommodationsstimulus $\Delta A_{Reiz}$ beeinflusst. Binokulares Sehen erhöht die Akkommo-dationsbreite $\Delta A^*_{max}$ um etwa 0,5 dpt.

Die Methode berücksichtigt lediglich die subjektiv bestimmte physiologische Akkommodationsbreite $\Delta A^*_{max}$ welche sich aus der Summe der tatsächliche Akkommodationsbreite $\Delta A_{max}$ und der Schärfentiefe T errechnet:

$$\Delta A^*_{max} = \Delta A_{max} + T \tag{2}$$

und die geschätzte Gebrauchsakkommodation $\Delta A_{Gebrauch}$ und ist daher sehr ungenau.

M. Millodot et al: " Presbyopia correction and the accommodation in reserve", in Ophthalmic & Physiological Optics UK, Bd. 9, Nr. 2, April 1989, Seiten 126-132 entnimmt man, dass sich die Addition Add mit Hilfe der Gleichung "Add = (1 metre/working distance in metres) - X (amplitude of accommodation)" bestimmen lässt, wobei X den genutzten Anteil der Akkommodationsbreite angibt. Das Dokument beschäftigt sich mit der Frage, welcher Anteil X der Akkommodationsbreite wohl tatsächlich genutzt wird. Zur Beantwortung dieser Frage wurden bei 305 Personen jeweils die Addition Add, die Akkommodationsbreite und die Lesedistanz bestimmt und aus diesen Größen jeweils mathematisch durch Umstellung der vorstehenden Gleichung der Anteil X der Akkommodationsbreite bestimmt. Als Addition Add wurde der entsprechende Wert der Testlinse gewählt, die der jeweilige Proband als am angenehmsten empfand. Die Lesedistanz wurde unmittelbar mit dem Metermaß gemessen. Die Akkommodationsbreite wurde mit Hilfe der "near point rule" gemessen. Dem Dokument entnimmt man, dass die gemessene Akkommodationsbreite bei über 52-jährigen Personen nicht der tatsächlichen Akkommodationsbreite entspricht, sondern der Schärfentiefe. Dies hält die Autoren jedoch nicht davon ab, den Anteil X im Mittel auf 49 % für Personen im Alter zwischen 53 und 62 Jahren bzw. 40 % für Personen im Alter zwischen 63 und 83 Jahren zu bestimmen.

C. T. Leffler et al. beschreiben in" Clinical predictors of the optimal spectacle correction for comfort performing desktop tasks", in Clinical and Experimental Optometry: Journal of the Australian Optometrical Association Nov. 2008, Bd. 91, Nr. 6, November 2008, Seiten 530 - 537, dass sich nach dem klassischen Modell die vorläufige Addition aus dem dioptrischen Arbeitsabstand minus der Hälfte der Akkommodationsbreite ergibt. Diese vorläufige Addition dient als Ausgangspunkt, der verfeinert werden muss, um die endgültige Addition zu erhalten. Fir die Bestimmung der Akkommodationsbreite werden verschiedene subjektive Verfahren vorgestellt.

### 4. Additionsbestimmung unter Berücksichtigung der relativen Akkommodation

Bei dieser Methode wird zunächst wiederum mit der Schätztabelle (Tabelle 1) unter Anwendung des unter 1. beschriebenen Verfahrens eine geeignete Addition $Add_{vorläufig}$ ermittelt. Dann wird eine geeignete Sehprobe (z.B. Duane'sche Strichfigur) in der Gebrauchsentfernung $a_{Gebrauch}$ fixiert. Die Addition $Add_{vorläufig}$ wird nun bis zu der Addition Add+ erhöht, bei der der Proband gerade noch scharf sieht. Danach wird die Addition $Add_{vorläufig}$ erniedrigt bis zu der Addition Add-, bei der der Proband gerade noch scharf sieht. Die Summe aus der stärksten Addition Add+ und der schwächsten Addition Add- dividiert durch 2 ergibt die notwendige Addition

$$Add = (Add+ + Add-)/2 \qquad (3)$$

Die Methode berücksichtigt allein das Alter, die Gebrauchsentfernung $a_{Gebrauch}$, die relative Akkommodation $\Delta A_{rel}$ und eine Abschätzung der Gebrauchsakkommodation $\Delta A_{Gebrauch}$ und ist daher ungenau.

Alle vorstehend beschriebenen Verfahren zur Bestimmung der Addition Add nach dem betriebsintern bekannten Stand der Technik weisen also Ungenauigkeiten auf, da sie auf Annahmen bzw. Schätzungen beruhen. Da alle genannten Verfahren subjektive Verfahren sind, unterliegen sie weiterhin den allgemeinen Nachteilen subjektiver Messverfahren (nämlich u.a. Validität der Aussagen des Probanden, Wahrnehmung des Probanden in der Messsituation etc.).

J. E. Wold et al. vergleichen in ihrem Aufsatz" Subjective and objective measurement of human accomodative amplitude", in Journal of Cataract and Refractive Surgery Oct. 2003, Bd. 29, Nr. 10, Oktober 2003, Seiten 1879-1888 verschiedene subjektive und objektive Verfahren zur Bestimmung der Akkommodationsbreite. Sie empfehlen die Verwendung objektiver Messungen der Akkommodation um festzustellen, ob die Akkommodation bei Alterssichtigen wiederhergestellt werden kann.

Die Aufgabe der Erfindung besteht daher darin, ein gegenüber dem Stand der Technik präziseres Verfahren zur Bestimmung der individuell erforderlichen Addition einer Sehhilfe und eine zur Durchführung des Verfahrens geeignete Vorrichtung bereitzustellen.

Diese Aufgabe wird durch ein Verfahren zur Bestimmung der individuell erforderlichen Addition einer Sehhilfe mit den Merkmalen des Patentanspruchs 1 sowie durch eine entsprechende Vorrichtung mit den Merkmalen des Patentanspruchs 9, ein Verfahren zur Bestimmung der individuell erforderlichen Addition einer Sehhilfe mit den Merkmalen des Patentanspruchs 3 sowie durch eine entsprechende Vorrichtung mit den Merkmalen des Patentanspruchs 15, einen Computer mit den Merkmalen des Patentanspruchs 8 sowie ein Computerprogramm mit den Merkmalen des Patentanspruchs 7 gelöst. Vorteilhafte Ausführungen und Weiterbildungen der Erfindung sind in den Unteransprüchen angegeben.

Die Erfinder haben festgestellt, dass die meisten der oben beschriebenen Verfahren auf dem Alter und dem Standardgebrauchsabstand $a_{Gebrauch}$ von 40 cm basieren und die tatsächliche Akkommodationsbreite $\Delta A_{max}$, die tatsächliche Schärfentiefe T als auch die Gewohnheiten Akkommodationsbreite $\Delta A_{max}$, die tatsächliche Schärfentiefe T als auch die Gewohnheiten des Probanden nicht oder nur unzureichend berücksichtigen. Diese Tatsache hat zur Folge, dass die Addition Add im Allgemeinen zu hoch angenommen wird. Eine unnötig hohe Addition Add verschlechtert das Design in Form von Verkleinerungen der nutzbaren Sehbereiche vor allem im Zwischen- und Nahbereich der Korrekturlinse mit den sich daraus ergebenden merkbaren Nachteilen für den Brillenträger.

Die Erfindung beruht nunmehr auf der Idee, die Schärfentiefe T des Auges zu berücksichtigen und/oder bislang lediglich abgeschätzte Eigenschaften des Auges der jeweiligen Probanden individuell zu bestimmen und anhand dieser vorzugsweise objektiv ermittelten Eigenschaften die erforderliche und am besten geeignete Addition Add der Sehhilfe für den Probanden zu ermitteln.

Ein erfindungsgemäßes Verfahren zur Bestimmung der individuell erforderlichen Addition Add einer Sehhilfe für ein Auge mit Hilfe eines Computers umfasst die folgenden Verfahrensschritte:

a) Bestimmung einer vorläufigen Addition $Add_{vorläufig}$ beispielsweise mit einem der vorstehend beschriebenen Verfahren,
b) Bestimmung der individuellen Schärfentiefe T des Auges,
c) Berechnung der Addition Add nach folgender Gleichung:

$$Add = Add_{vorläufig} - \omega T, \qquad (4)$$

wobei $\omega$ eine reelle Zahl darstellt, die im Bereich $0 < \omega \leq 1$ liegt. Üblicherweise liegen die Werte für $\omega$ im Bereich zwischen $^1/_4 \leq \omega \leq {}^3/_4$. Geeignete Werte für den Faktor $\omega$ zur Gewichtung der Schärfentiefe T sind also z.B. 0,5 oder 2/3.

Die entsprechende Vorrichtung zur Durchführung des Verfahrens umfasst demgemäß folgende Komponenten:

a) eine Additionsbestimmungseinrichtung zur Bestimmung einer vorläufigen Addition $Add_{vorläufig}$ für ein Auge eines Probanden
b) eine Schärfentiefenbestimmungseinrichtung zur Bestimmung der individuellen Schärfentiefe T des Auges des Probanden und
c) eine Berechnungseinrichtung zur Berechnung der Addition Add nach der obigen Gleichung (4).

Ein weiteres erfindungsgemäßes Verfahren zur Bestimmung der Individuen erforderlichen Addition Add einer Sehhilfe für ein Auge mit Hilfe eines Computers umfasst folgende Verfahrensschritte:

a) die Akkommodationsbreite $\Delta A_{max}$ wird individuell und objektiv bestimmt,
b) die Gebrauchsentfernung gebrauch wird individuell bestimmt,
c) die Addition Add wird nach folgender Gleichung berechnet:

$$Add = 1/a_{Gebrauch} - \sigma\Delta A_{max}, \qquad\qquad (5)$$

wobei $\sigma$ eine reelle Zahl darstellt, die im Bereich $0<\sigma\leq 1$ liegt. Meist liegen die Werte für $\sigma$ im Intervall $^1/_4\leq\sigma\leq^3/_4$. $\sigma$ kann also z.B. $^1/_2$, $^2/_3$ oder $^5/_{12}$ sein.

Eine entsprechende erfindungsgemäße Vorrichtung umfasst

a) eine Akkommodationsbreitenbestimmungseinrichtung zur individuellen und objektiven Bestimmung der Akkommodationsbreite $\Delta A_{max}$,
b) eine Gebrauchsentfernungsbestimmungseinrichtung zur individuellen Bestimmung der Gebrauchsentfernung $a_{Gebrauch}$ und
c) eine Additionsberechnungseinrichtung zur Berechnung der Addition Add nach vorstehender Gleichung (5).

Die oben angegebenen erfindungsgemäßen Verfahren können z.B. auf einem Computer ausgeführt werden, der zur Ausführung des jeweiligen Verfahrens entsprechend eingerichtet ist.

Ein Computerprogramm bzw. ein Computerprogrammprodukt mit Programmcode kann zur Ausführung des jeweiligen Verfahrens auf einem Computer eingerichtet sein. Das Computerprogramm kann auf einem maschinenlesbaren Datenträger abgespeichert sein.

Die vorläufige Addition $Add_{vorläufig}$ kann z.B. anhand einer Schätzung der physiologischen Akkommodationsbreite $\Delta A^*_{max}$ bestimmt werden. Beispielsweise kann das vorstehend beschriebene Diagramm nach Duane verwendet werden. Die Berechnung der vorläufigen Addition $Add_{vorlaufig}$ kann z.B. mit Hilfe eines der vorstehend beschriebenen Verfahren 1 oder 2 erfolgen. Diese Verfahren zur Berechnung der vorläufigen Addition $Add_{vorlaufig}$ zeichnen sich meist durch deren Bekanntheit bei den anwendenden Optikern aus. Eine Schulung der Optiker.zur. Anwendung des erfindungsgemäßen Verfahrens kann daher weitgehend entfallen.

Die vorläufige Addition $Add_{vorläufig}$ kann auch anhand einer Schätzung der tatsächlichen Akkommodationsbreite $\Delta A_{max}$ bestimmt werden. Eine Schätzung der tatsächlichen Akkommodationsbreite $\Delta A_{max}$ kann z.B. darin bestehen, dass der Proband mit Fernkorrektion eine geeignete Leseprobe so nah zum Auge führt, bis die Leseprobe gerade unscharf erscheint. Der Kehrwert des in Meter gemessenen Abstandes $a_{Gebrauch}$ der Leseprobe zum Hauptpunkt $H_A$ des Auges des Probanden ergibt die Akkommodationsbreite $\Delta A_{max}$.

Eine Schätzung der Gebrauchsakkommodation $\Delta A_{Gebrauch}$ kann ebenfalls als Grundlage der Bestimmung der vorläufigen Akkommodation $Add_{vorläufig}$ dienen. Die Gebrauchsakkommodation $\Delta A_{Gebrauch}$ wird z.B. nach Reiner zur Hälfte der Akkommodationsbreite $\Delta A_{max}$ angenommen. Schober nimmt diese zu 2/3 der Akkommodationsbreite $\Delta A_{max}$ an.

Die vorläufige Addition $Add_{vorläufig}$ kann z.B. auch anhand einer individuellen Messung der physiologischen Akkommodationsbreite $\Delta A^*_{max}$ berechnet werden. Das in der Beschreibungseinleitung unter 3. beschriebene Beispiel stellt einen Vorschlag zur Bestimmung der vorläufigen Addition $Add_{vorläutig}$ nach dieser Methode dar.

Die Bestimmung der physiologischen Akkommodationsbreite $\Delta A^*_{max}$ kann z.B. auch dadurch erfolgen, dass der Nahpunkt in der Entfernung $a_P$ mittels Heranführen einer geeigneten Testfigur durch den für die Ferne vollkorrigierten Probanden monokular ermittelt wird. Da der Nahpunkt bekanntermaßen die höchste Anspannung der Akkommodation fordert, ist es nötig, die Messung möglichst schnell durchzuführen, nämlich innerhalb der Akkommodationszeit ($t < t_A$). Ansonsten läuft man Gefahr, nicht die physiologische Akkommodationsbreite $\Delta A^*_{max}$ als vielmehr die Gebrauchsakkommodation $\Delta A_{Gebrauch}$ zu messen. Die physiologische Akkommodationsbreite $\Delta A^*_{max}$ ergibt sich dann aus dem Kehrwert des in Meter gemessenen Nahpunktabstandes $a_P$:

$$\Delta A^{\bullet}_{max} = 1/a_p \qquad\qquad (6)$$

Eine andere Möglichkeit besteht darin eine geeignete Testfigur auf eine Einstellentfernung $a_E$ von z.B. 40 cm (entsprechend einer Einstellpunktrefraktion $A_E$ von 2,5 dpt) zum Augenhauptpunkt $H_A$ des Probanden zu positionieren. Sieht der Proband die Testfigur scharf, werden solange binokular negative sphärische Gläser vorgegeben, bis die Testfigur-gerade so verschwimmt. Sieht der Proband die Testfigur nicht scharf, werden solange binokular positiv sphärische Gläser vorgegeben, bis die Testfigur gerade so scharf erscheint. Der mit entweder negativen oder positiven sphärischen Gläsern ermittelte Wert $S'_{binokular}$ der sphärischen Wirkung des so ermittelten Glases wird notiert. Die physiologische Akkommodationsbreite $\Delta A^*_{max}$ ergibt sich dann aus der Gleichung:

$$\Delta A^{\bullet}_{max} = A_E - S'_{binokular}, \tag{7}$$

mit $A_E$ = 2,5 dpt. Ein Nachteil dieser Möglichkeit stellt die durch die binokulare Messsituation Konvergenz hervorgerufene dar.

Eine weitere Möglichkeit, die physiologische Akkommadationsbreite $\Delta A^*_{max}$ zu bestimmen, wird im Folgenden beschrieben:

Anhand der Kurve aus Figur 16 wird dem Probanden eine geeignete Testfigur in entsprechendem Abstand $a_{Gebrauch}$ (entspricht der für das Alter des Probanden durchschnittlichen minimalen Sehweite $a_P$) vorgehalten. Wird die Testfigur dort scharf erkannt, werden solange monokular negative sphärische Gläser vorgegeben, bis die Testfigur gerade verschwimmt. Wird die Testfigur dort nicht scharf erkannt, werden solange monokular positive sphärische Gläser vorgegeben, bis die Testfigur gerade so verschwimmt. Die individuelle physiologische Akkommodationsbreite $\Delta A^*_{max}$ ergibt sich dann aus dem Kehrwert der durchschnittlichen minimalen Sehweite $a_{PDuane}$ (entspricht der durchschnittlichen physiologischen Akkommodationsbreite $\Delta A^*_{max}$ aus der mittleren Kurve 802 des Diagramms nach der Figur 15) und dem ermittelten Wert der vorgegebenen Gläser $S'_{monokular}$. Dazu gilt folgende Gleichung:

$$\Delta A^{\bullet}_{max} = \Delta A^{\bullet}_{max, m,} - S'_{monokular}, \tag{8}$$

wobei

$$\Delta A^{\bullet}_{max, m,} = 1/a_{PDuane} \tag{9}$$

ist.

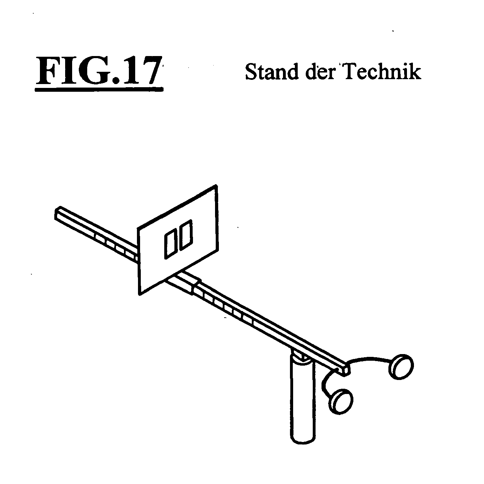

In den drei vorstehend im Detail beschriebenen Varianten kann ein Akkommodometer wie in Figur 17 dargestellt verwendet werden (aus Heinz Diepes, "Refraktionsbestimmung", Verlag Heinz Postenrieder, Pforzheim, 2. Auflage, 1975, S. 414).

Es kann auch eine individuelle Messung der tatsächlichen Akkommodationsbreite $\Delta A^*_{max}$ durchgeführt werden. Aus der US 6,554,429 B1 ist es mittlerweile z.B. bekannt, die tatsächliche Akkommodationsbreite $\Delta A_{max}$ mit Hilfe einer Wellenfrontmessung objektiv zu bestimmen. Eine Durchführung der Methode unter Berücksichtigung der objektiv bestimmten Akkommodationsbreite $\Delta A_{max}$ verbessert die Genauigkeit signifikant.

Schließlich kann auch eine Messung der tatsächlichen individuellen Gebrauchsakkommodation $\Delta A_{Gebrauch}$ durchgeführt und anhand dieser wiederum die vorläufige Addition Add$_{vorläufig}$ bestimmt werden. Ein Beispiel stellt das in der Beschreibungseinleitung unter 4. beschriebene Verfahren dar.

Für die Bestimmung der Gebrauchsakkommodation $\Delta A_{Gebrauch}$ kann dem für die Ferne vollkorrigierten Probanden auch eine geschätzte Addition Add$_{geschätzt}$ in die Messbrille eingesetzt werden. Der Proband wird nun aufgefordert, eine geeignete Testfigur monokular so nah zum Auge heranzuführen, bis er die Testfigur gerade noch scharf erkennen kann. Dies entspricht dem Nahpunktabstand $a_P$. Nun wird der Proband aufgefordert, so lange monokular auf die Testfigur zu blicken, bis diese aufgrund der nachlassenden Akkommodation $\Delta A$ unscharf erscheint. In einem nächsten Schritt soll der Proband nun die Testfigur so weit von sich weg halten, bis diese wieder scharf erscheint (entspricht dem Einstellpunkt $a_E$). Der Kehrwert des in Meter gemessenen Abstandes $a_E$ zum Einstellpunkt ergibt die Gebrauchsakkommodation $\Delta A_{Gebrauch}$:

$$\Delta A_{Gebrauch} = 1/a_E \tag{10}$$

In einer besonderen Ausgestaltung der Erfindung kann auch eine Gebrauchsentfernung $a_{Gebrauch}$ bestimmt werden und die vorläufige Addition Add$_{vorläufig}$ nach folgender Gleichung berechnet werden:

$$Add_{vorläufig} = 1/a_{Gebrauch} - \sigma \Delta A_{max}, \tag{11}$$

wobei $\sigma$ eine reelle Zahl darstellt, die im Bereich $0 \le \sigma \le 1$ liegt. Da die vorläufige Addition $\text{Add}_{\text{vorläufig}}$ eine reine Rechengröße darstellt, kann diese tatsächlich auch mit dem Kehrwert des Gebrauchsabstands $a_{\text{Gebrauch}}$ gleichgesetzt werden.

Die Gebrauchsentfernung $a_{\text{Gebrauch}}$ kann z.B. individuell bestimmt werden, indem einem Probanden ein Gegenstand dargeboten wird und als Gebrauchsentfernung $a_{\text{Gegebrauch}}$ der Abstand vom objektseitigen Hauptpunkt $H_A$ des Auges zu dem Ort angenommen wird, an dem sich der Gegenstand beim entspannten Betrachten durch den Probanden befindet. Dabei ist die individuelle Bestimmung einer Gebrauchsentfernung $a_{\text{Gebrauch}}$ des Probanden nicht grundsätzlich auf die gewünschte Leseentfernung beschränkt, sondern kann auch für alle anderen gewünschten Entfernungen, wie z.B. Arbeit an einem Rechner mit Monitor, bestimmt werden.

Die Gebrauchsentfernung $a_{\text{Gebrauch}}$ kann auch durch Anamnese mit Angabe der Hauptsehaufgaben und den dazugehörigen Entfernungen durch den Probanden bestimmt werden. Beide o.a. Methoden stellen subjektive Verfahren dar, die sich aber durch geeignete Fragestellungen an den Probanden bis zu einem gewissen Grad objektivieren lassen.

Häufig ist es jedoch präziser, die Gebrauchsentfernung gebrauch objektiv individuell zu bestimmen. Objektiv kann die Gebrauchsentfernung $a_{\text{Gebrauch}}$ z.B. dadurch ermittelt werden, dass eine Messvorrichtung eine oder verschiedene für den Probanden wichtige Gebrauchsentfernungen $a_{\text{Gebrauch}}$ automatisch misst und gegebenenfalls mittelt. Die WO 2008/064379 A1 beschreibt beispielsweise eine Vorrichtung zum Erfassen der Lese-Sehschärfe mit der sich auch die Gebrauchsentfernung $a_{\text{Gebrauch}}$ bestimmen lässt. Es sind Mittel vorgesehen, die den vom Probanden frei wählbaren Leseabstand $a_{\text{Gebrauch}}$ von dem dargestellten Text oder der dargestellten Graphik auf einer Präsentationsfläche messen.

Die Akkommodationsbreite $\Delta A_{\text{max}}$ kann mit Hilfe unterschiedlichster Geräte individuell und objektiv bestimmt werden. Die tatsächliche Akkommodationsbreite $\Delta A_{\text{max}}$ kann -wie oben bereits angedeutet wurde- aus einer Wellenfrontmessung bestimmt werden. So kann z.B. zur Bestimmung der Akkommodationsbreite $\Delta A_{\text{max}}$ eine Wellenfrontmesseinrichtung, d.h. ein Wellenfrontsensor wie z.B. ein Shack-Hartmann-Sensor, zum Einsatz kommen. Ein Verfahren zur Bestimmung der Akkommodationsbreite $\Delta A_{\text{max}}$ unter Zuhilfenahme eines Wellenfrontsensors ist -wie oben bereits angedeutet wurde - z.B. dem Dokument US 6,554,429 B1 (siehe insbesondere Spalte 3, Zeile 23 bis Spalte 4, Zeile 25 und Anspruch 1) zu entnehmen. Dort sind auch unterschiedliche Arten geeigneter Sensoren angegeben (siehe dort Spalte 3, Zeilen 10-13).

Die tatsächliche Akkommodationsbreite $\Delta A_{\text{max}}$ kann jedoch alternativ auch als Differenz zwischen der aus einer Wellenfrontmessung oder einer Autorefraktionsmessung des Auges objektiv bestimmten Fernpunktrefraktion $A_R$ und der aus einer Wellenfrontmessung oder einer Autorefraktionsmessung des Auges objektiv bestimmten Nahpunktrefraktion $A_P$ bestimmt werden.

Konkret kann diese Bestimmung der tatsächlichen Akkommodationsbreite $\Delta A_{\text{max}}$ beispielsweise in folgenden Schritten erfolgen:

a) Eine an der Retina reflektierte Wellenfront eines auf das nicht akkommodierende Auge treffenden Lichtstrahls mit vorbestimmter Wellenfront oder eine Autorefraktion des nicht akkommodierenden Auges wird gemessen. Vorzugsweise wird dem Auge dabei kein Akkommodationsreiz $\Delta A_{\text{Reiz}}$ dargeboten.

b) Aus der in Schritt a) gemessenen Wellenfront oder der in Schritt a) gemessenen Autorefraktion wird die Fernpunktrefraktion $A_R$ des Auges berechnet.

c) Daraufhin wird ein die tatsächliche Akkommodationsbreite $\Delta A_{\text{max}}$ übersteigender Akkommodationsreiz $\Delta A_{\text{Reiz}}$ für das Auge dargeboten.

d) Dann wird eine an der Retina reflektierte Wellenfront eines auf das aufgrund des Akkommodationsreizes $\Delta A_{\text{Reiz}}$ akkommodierenden Auge treffenden Lichtstrahls mit vorbestimmter Wellenfront oder eine Autorefraktion des aufgrund des Akkommodationsreizes $\Delta A_{\text{Reiz}}$ akkommodierenden Auges gemessen. Konkret erfolgt dies dadurch, dass die Fixationsentfernung $a_E$, also die Strecke vom Augenhauptpunkt $H_A$ zum Einstellpunkt des Auges, so gewählt wird, dass das Auge nicht mehr in der Lage ist, das sich im Fixationspunkt befindliche Objekt scharf wahrzunehmen.

e) Aus der in Schritt d) gemessenen Wellenfront oder der in Schritt d) gemessenen Autorefraktion wird die tatsächliche Einstellpunktrefraktion $A_E$ des Auges berechnet.

f) Die in Schritt e) berechnete tatsächliche Einstellpunktrefraktion $A_E$ wird mit der nachfolgend als ideal bezeichneten Einstellpunktrefraktion $A_{E,\text{ideal}}$ verglichen, die sich ergäbe, wenn die Akkommodation A des Auges dem Akkommodationsreiz $\Delta A_{\text{Reiz}}$ folgen könnte.

g) Danach wird der Akkommodationsreiz $\Delta A_{\text{Reiz}}$ kontinuierlich oder in diskreten Schritten von z.B. 0.05 dpt verringert und fortlaufend entsprechend den Schritten e) und f) die tatsächliche Einstellpunktrefraktion $A_E$ gemessen und mit der idealen Einstellpunktrefraktion $A_E$ verglichen.

h) Als Nahpunktrefraktion $A_P$ wird dann z.B. die tatsächliche Einstellpunktrefraktion $A_E$ gewählt, die bei dem unter Schritt g) durchgeführten Vergleich erstmalig der Änderung der idealen Einstellpunktrefraktion $A_E$ folgt.

Alternativ ist es möglich, lediglich im Anschluss an den Schritt e) den Akkommodationsreiz $\Delta A_{\text{Reiz}}$ kontinuierlich oder in diskreten Schritten zu verringern und fortlaufend entsprechend den Schritten e) und f) die tatsächliche Einstellpunktrefraktion $A_E$ zu messen und als Nahpunktrefraktion $A_P$ die gemessene tatsächliche Einstellpunktrefraktion $A_E$

(bzw. eine der gemessenen tatsächlichen Einstellpunktrefraktionen bzw. einen Mittelwert der gemessenen tatsächlichen Einstellpunktrefraktionen) zu verwenden, bei der die gemessene Einstellpunktrefraktion $A_E$ dem Akkommodationsreiz $\Delta A_{Reiz}$ nicht mehr folgt.

Weiter alternativ ist es möglich, anstelle des Verfahrensschritts c) dem Auge einen Akkommodationsreiz $\Delta A_{Reiz}$ darzubieten, dem dieses problemlos folgt und dann den Akkommodationsreiz $\Delta A_{Reiz}$ kontinuierlich oder in diskreten Schritten so lange (und ggf. etwas darüber hinaus) zu erhöhen, bis die Akkommodation A des Auges diesem Reiz $DA_{Reiz}$ nicht mehr folgen kann. Die Verfahrensschritte d) bis h) können dann z.B. in entsprechender Weise abgearbeitet werden. Als Nahpunktrefraktion $A_P$ wird dann z.B. die tatsächliche Einstellpunktrefraktion $A_E$ gewählt, die bei dem unter Schritt g) durchgeführten Vergleich letztmalig der Änderung der idealen Einstellpunktrefraktion $A_E$ ideal folgt. Selbstverständlich kann auch als Nahpunktrefraktion $A_P$ die gemessene tatsächliche Einstellpunktrefraktion $A_E$ (bzw. eine der gemessenen tatsächlichen Einstellpunktrefraktionen bzw. ein Mittelwert der gemessenen tatsächlichen Einstellpunktrefraktionen $A_E$) verwendet werden, bei der die gemessene Einstellpunktrefraktion $A_E$ dem Akkommodationsreiz $\Delta A_{Reiz}$ gerade nicht (mehr) folgt.

Die vorläufige Addition $Add_{vorläufig}$ kann z.B. nach erfolgter Bestimmung der Gebrauchsentfernung $a_{Gebrauch}$ nach folgender Gleichung berechnet werden:

$$Add_{vorläufig} = 1/a_{Gebrauch} - \Delta A_{Gebrauch}. \qquad (12)$$

Die tatsächliche Gebrauchsakkommodation $\Delta A_{Gebrauch}$ kann z.B. wie folgt gemessen werden:

Es wird die gleiche Messung wie bereits bzgl. der Messung der Akkommodationsbreite beschrieben durchgerührt, nur mit dem Unterschied, dass die Messung länger dauert und der jeweilige Akkommodationsreiz $\Delta A_{Reiz}$ dem Auge mehrere Sekunden dargeboten wird, und zwar idealerweise so lange, bis die Akkommodation A dem Reiz $\Delta A_{Reiz}$ vollständig gefolgt ist. Die Dauer t des Akkommodationsreizes $\Delta A_{Reiz}$ ist also größer als die Akkommodationszeit $t_A$ gewählt.

Für die Bestimmung der Gebrauchsakkommodation $\Delta A_{Gebrauch}$ wird der für die Ferne vollkorrigierte Proband aufgefordert, eine geeignete Testfigur monokular so nah zum Auge heranzuführen, bis er die Testfigur gerade noch scharf erkennen kann. Diese Testfigur kann sich beispielsweise auf einem Akkommodometer, wie in Figur 17 dargestellt, befinden. Der so ermittelte Abstand entspricht dem Nahpunktabstand $a_P$. Reicht aufgrund hochgradiger Presbyopie die Länge der Akkommodometerschiene (oder, wenn die Messung ohne Akkommodometer vorgenommen wird, die Armlänge des Probanden) zur Messung nicht aus, so setzt der Untersucher auf Verdacht einen sphärischen Nahzusatz vor, der später von der gemessenen Gebrauchsakkommodation $\Delta A_{Gebrauch}$ subtrahiert wird. Nun wird der Proband aufgefordert, so lange monokular auf die Testfigur zu blicken, bis diese aufgrund der nachlassenden Akkommodation $\Delta A$ unscharf erscheint. In einem nächsten Schritt soll der Proband nun die Testfigur so weit von sich weg halten, bis diese wieder scharf erscheint (entspricht dem Einstellpunkt $a_E$). Der Kehrwert des in Meter gemessenen Abstandes zum Einstellpunkt $a_E$ ergibt die Gebrauchsakkommodation $\Delta A_{Gebrauch}$.

Die vorläufige Addition $Add_{vorläufig}$ kann z.B. auch nach herkömmlicher Methode aus der Fernpunktrefraktion $A_R$ und der Nahpunktrefraktion $A_P$ nach folgender Gleichung berechnet werden:

$$Add_{vorläufig} = A_p - A_R \qquad (13)$$

Die Schärfentiefe T entspricht gerade dem zweifachen des maximalen Akkommodationsreizes $\Delta A_{Reiz}$ an dem das Auge gerade noch nicht akkommodiert.

Die Verwendung einer Wellenfrontmesseinrichtung zur Bestimmung der Schärfentiefe T ist hier zwingend erforderlich. Die Verwendung einer Autorefraktormesseinrichtung ist zur Bestimmung der Schärfentiefe T alleine nicht möglich. Bei Aphakie oder Pseudophakie ist nur die letztgenannte Methode zur Bestimmung der Schärfentiefe T auf Basis einer Messung mit einer Wellenfrontmesseinrichtung möglich.

Die individuelle Schärfentiefe T des Auges eines Probanden lässt sich z.B. aus einer Sequenz von Wellenfrontmessungen oder einer Sequenz von Autorefraktionsmessungen bei Fixationsentfernungen in der näheren Umgebung des Fernpunkts bestimmt. Als maximalen Akkommodationsreiz $\Delta A_{Reiz}$ nimmt man z.B. den Akkommodationsreiz $\Delta A_{Reiz}$ (entsprechend dem Kehrwert der Fixationsentfernung), bei dem die gemessene Einstellpunktrefraktion $A_E$ gerade von der aus einer Wellenfrontmessung oder einer Autorefraktionsmessung ermittelten Fernpunktrefraktion $A_R$ abweicht oder gerade noch der entsprechenden Fernpunktrefraktion $A_R$ entspricht. Als maximalen Akkommodationsreiz $\Delta A_{Reiz}$ kann man auch den Akkommodationsreiz $\Delta A_{Reiz}$ nehmen, bei dem die gemessene Wellenfront gerade noch mit der Wellenfront bei unendlicher Fixationsentfernung übereinstimmt oder bei dem die gemessene Wellenfront gerade geringfügig von der Wellenfront bei unendlicher Fixationsentfernung abweicht.

Ein weiteres Verfahren zur Bestimmung der Schärfentiefe T auf Basis einer Wellenfrontanalyse kann z.B. folgende Verfahrensschritte umfassen:

a) Eine an der Retina reflektierten Wellenfront eines auf das nicht akkommodierende Auge treffenden Lichtstrahls mit vorbestimmter Wellenfront oder eine Autorefraktion des nicht akkommodierenden Auges wird gemessen. Vorzugsweise wird dem Auge dabei kein Akkommodationsreiz dargeboten.

b) Vorher, gleichzeitig oder danach wird der Pupillendurchmesser des Auges gemessen. Wesentlich dabei ist, dass der Pupillendurchmesser im Zustand nach Schritt a) gemessen wird. Die Messung des Pupillendurchmessers kann z.B. dadurch erfolgen, dass von dem Auge eine Kameraaufnahme gemacht wird und der Durchmesser der Pupille aus der Aufnahme entnommen wird.

c) Daraufhin erfolgt die Umrechnung der gemessenen Wellenfront auf einen gewünschten Pupillendurchmesser. J. Schwiegerling hat in seinem Artikel "Scaling Zernike expansion coefficients to different pupil sizes", welcher in J. Opt. Soc. Am. A, Vol. 19, No. 10 auf den Seiten 1937 bis 1945 veröffentlicht wurde, Möglichkeiten zur Umrechungen aufgezeigt.

d) In einem weiteren Schritt wird ein Fixationstarget mit einer Darstellung von Strukturen mit vorgegebenen räumlichen Frequenzen entsprechend den gewünschten Anforderungen, z.B. Visus V = 0,4 beim Lesen, bereitgestellt.

e) Danach erfolgt die Festlegung eines Suchraumes von z.B. +/-5 dpt oder +/-3 dpt um die mittlere Sphäre mSph = der Fernpunktrefraktion $A_R$ in z.B. 0.05 dpt oder 0.1 dpt Schritten, wobei

$$\mathrm{mSph} = Sph + \frac{Zyl}{2} \qquad\qquad (14)$$

mit Sph der sphärischen Wirkung und Zyl der astigmatischen Wirkung einer für den Probanden vorgesehenen Sehhilfe.

f) Daraufhin erfolgt eine Faltung der Wellenfront um die vom jeweiligen Punkt im Suchraum vorgegebene mittlere Sphäre $mSph_{korr}$ Diese korrigierte Wellenfront wird anschließend mit den Strukturen des Fixationstargets gefaltet. Diese Faltung dient der Simulation des Seheindrucks des Probanden mit im Suchraum geänderter Wellenfront.

g) Die nachfolgende Auswertung der Faltung besteht z.B. in der Ermittlung des Verhältnisses Q aus den maximalen und minimalen Intensitäten $I_{max}$, $I_{min}$ der durch die Faltung entstandenen Strukturen:

$$Q = \frac{I_{max} - I_{min}}{I_{max}} \qquad\qquad (15)$$

h) Die Schritte f) und g) werden nun für alle Werte $mSph_{korr}$ innerhalb des Suchraumes durchgeführt. Somit erhält man für jedes $mSph_{korr}$ einen Wert für das Verhältnis Q.

Als Schärfentiefe T wird dann der Betrag der Differenz der Werte der mittleren Sphäre $mSph_{korr}$ im Suchraum angenommen, bei dem das Verhältnis Q ein vorgegebenes Verhältnis $Q_{vor}$ gerade über- bzw. unterschreitet. Max Born et al geben "Principles of Optics", Cambridge University Press, 7th expanded edition, S. 370-371 z.B. als Schwelle bei der nebeneinander liegende Strukturen gerade noch getrennt wahrnehmbar sind, ein Verhältnis Q der Intensitätsdifferenz $I_{max} - I_{min}$ zur maximalen Intensität $I_{max}$ von 19% an (sogenanntes Rayleigh Kriterium):

$$Q = \frac{I_{max} - I_{min}}{I_{max}} = 0,19 \qquad\qquad (16)$$

Ein anderes Verfahren zur Bestimmung der Schärfentiefe T auf Basis einer Wellenfrontanalyse kann folgende Verfahrensschritte umfassen:

a) Eine an der Retina reflektierte Wellenfront eines auf das nicht akkommodierende Auge treffenden Lichtstrahls mit vorbestimmter Wellenfront oder eine Autorefraktion des nicht akkommodierenden Auges wird gemessen. Vorzugsweise wird dem Auge dabei kein Akkommodationsreiz $\Delta A_{Reiz}$ dargeboten.

b) Vorher, gleichzeitig oder danach wird der Pupillendurchmesser des Auges gemessen (vgl. das vorstehend beschriebene Verfahren).

## EP 2 208 457 B1

c) Daraufhin erfolgt eine Umrechnung der gemessenen Wellenfront auf einen gewünschten Pupillendurchmesser.

d) Im Weiteren erfolgt eine Bestimmung der Kaustik im Bereich von z.B. +/-3dpt um die mittlere Sphäre mSph der Fernppunktrefrakion $A_R$. Unter Kaustik versteht man die Einhüllende der Strahlen im Bildraum bei sphärischer Aberration (vgl. Helmut Goersch, Zeiss Handbuch für Augenoptik, Auflage 2000, Seite 35).

e) Danach erfolgt eine Summation der Intensitäten an jeder Stelle im Bereich von z.B. +/-3 dpt um die mittlere Sphäre mSph der Kaustik in einem Zylindervolumen um die optische Achse oA.

f) Die Bestimmung der Schärfentiefe T erfolgt dann aus dem Abstand der beiden Punkte entlang der Kaustik, an denen die Intensität um eine bestimmte Schwelle, z.B. 19%, kleiner ist, als die maximale Intensität.

Die Addition Add, $Add_{vorläufig}$ nach der Erfindung wird (im Allgemeinen zunächst) monokular bestimmt. Binokular lässt sich die Addition Add, $Add_{vorläufig}$ dadurch bestimmen, dass z.B. für die Sehhilfen beider Augen die gleiche Addition Add, $Add_{vorinurig}$ verwendet wird. Es kann dabei z.B. der Mittelwert der jeweils monokular ermittelten Additionen Add, $Add_{vorläufig}$ gewählt werden. Alternativ kann z.B. für die Sehhilfen beider Augen die größere der für die beiden Sehhilfen ermittelten Additionen Add, $Add_{vorläufig}$ verwendet werden.

Die Erfindung wird im Folgenden anhand der Zeichnungen näher beschrieben. Es zeigen:

Figur 1   ein Flussdiagramm aus dem sich die einzelnen Verfahrensschritte eines Ausführungsbeispiels eines erfindungsgemäßen Verfahrens zur Bestimmung der individuell erforderlichen Addition Add einer Sehhilfe für einen Probanden entnehmen lassen,

Figur 2   eine Vorrichtung, welche zur Bestimmung der individuell erforderlichen Addition Add nach dem in der Figur 1 skizzierten Verfahren geeignet ist,

Figur 3   ein Flussdiagramm aus dem sich die einzelnen Verfahrensschritte eines weiteren Ausführungsbeispiels eines erfindungsgemäßen Verfahrens zur Bestimmung der individuell erforderlichen Addition Add einer Sehhilfe für einen Probanden entnehmen lassen,

Figur 4   ein Flussdiagramm eines Beispiels für die Bestimmung der individuellen Gebrauchsentfernung $a_{Gebrauch}$ eines Probanden,

Figur 5   ein Flussdiagramm eines Beispiels für die Bestimmung der individuellen Akkommodationsbreite $\Delta A_{max}$ und der Schärfentiefe T eines Auges eines Probanden,

Figur 6   die gemessene mittlere sphärische mSph des Auges eines Probanden in Abhängigkeit vom Akkommodationsreiz $\Delta A_{Reiz,}$

Figur 7   ein Flussdiagramm eines weiteren Beispiels für die Bestimmung der individuellen Akkommodationsbreite $\Delta A_{max}$ und der Schärfentiefe T eines Auges eines Probanden,

Figur 8   ein Flussdiagramm eines Beispiels für die Bestimmung der individuellen Schärfentiefe T des Auges eines Probanden,

Figur 9   ein Ausführungsbeispiel für ein Fixationstarget zur Durchführung des Verfahrens nach dem Flussdiagramm nach Figur 8,

Figur 10   schematische Intensitätsprofile als Maß für die Wahrnehmung zweier benachbarter Linien des Fixationstargets nach der Figur 9, wenn sich das Fixationstarget im Fernpunkt $a_R$ und in unterschiedlichen Abständen zum Fernpunkt befindet,

Figur 11   ein Flussdiagramm eines zweiten Beispiels für die Bestimmung der individuellen Schärfentiefe T des Auges eines Probanden,

Figur 12   einen Längsschnitt durch die Kaustik eines durch das Auge eines Probanden tretenden Strahlbündels mit eingangsseitig ebener Wellenfront im Bereich der Netzhaut,

Figur 13   die in einem Zylinder im Bereich der Kaustik nach der Figur 12 eingeschlossene Lichtintensität in Abhängigkeit des Abstandes $a_{NH}$ von der Netzhautebene,

**12**

Figur 14    ein Diagramm, in dem die Akkommodation A eines Auges unterschiedlicher Prebanden über dem Akkommodationsanreiz $\Delta A_{Reiz}$ aufgetragen ist:

    A: individuelle Gebrauchsakkommodation $\Delta A_{Gebrauch}$, 50, eines 50 jährigen
    B: individuelle Akkommodationsbreite $\Delta A_{max,\ 50}$ eines 50 jährigen
    C: mittlere Akkommodationsbreite $\Delta A^{*}_{max,m,\ 50}$ eines 50 jährigen nach Duane
    D: mittlere Akkommodationsbreite $\Delta A^{*}_{max,\ m,\ 30}$ eines 30 jährigen nach Duane
    E: ideale Akkommodationsbreite $\Delta A_{max}$

Figur 15    die Altersabhängigkeit der durchschnittlichen physiologischen Akkommodationsbreite $\Delta A^{*}_{max}$ nach Duane (Stand der Technik),

Figur 16    die Altersabhängigkeit der durchschnittlichen Sehweite (=Nahpunktabstand) $a_p$ nach dem Stand der Technik,

Figru 17    Akkommodometer (Stand der Technik).

<u>Ausführungsbeispiel zur Bestimmung der Addition</u>

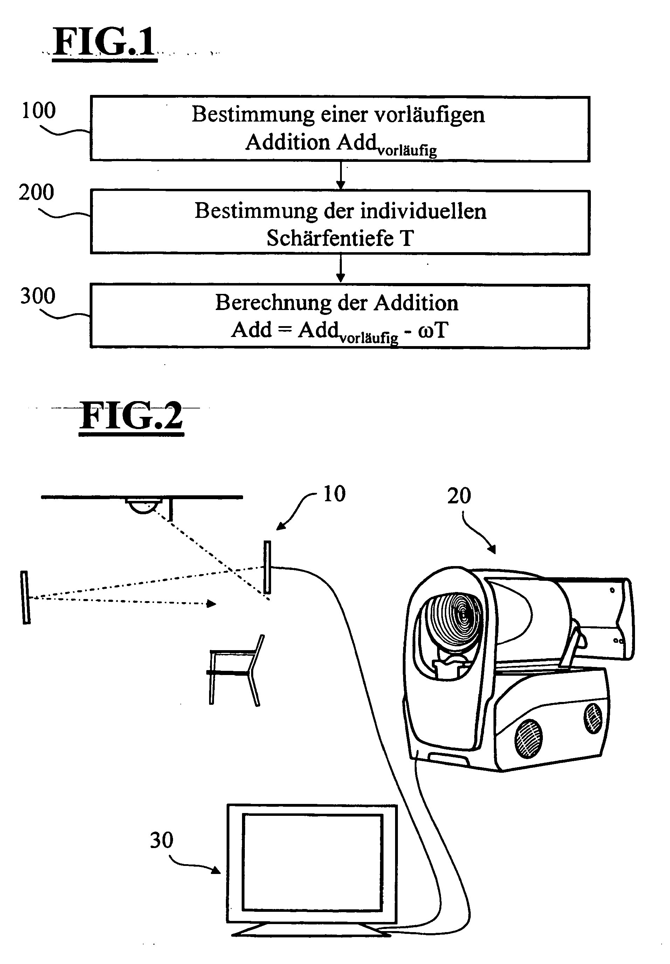

Die Figur 1 zeigt ein Flussdiagramm aus dem sich die einzelnen Verfahrensschritte einer Variante eines erfindungsgemäßen Verfahrens zur Bestimmung der individuell erforderlichen Addition Add einer Sehhilfe für einen Probanden entnehmen lassen. Die Figur 2 zeigt eine entsprechende Vorrichtung zur Durchführung des Verfahrens. Einzelheiten zum Verfahren sowie zur Vorrichtung werden nachfolgend beschrieben.

In einem ersten Schritt 100 wird eine vorläufige Addition $Add_{vorlaufig}$ für die Sehhilfe (z.B. Brillenlinse) eines Auges des Probanden bestimmt. Als Verfahren zur Bestimmung der vorläufigen Addition $Add_{vorläufig}$ kann eines der vorstehend in der Beschreibungseinleitung unter 1. bis 4. beschriebenen und aus dem Stand der Technik bekannten Verfahren zur Anwendung kommen. Beispielsweise kann auch ein Verfahren verwendet werden, bei dem der Proband aus einer Mehrzahl an Testbrillen die für ihn günstigste auswählt. Es ist auch möglich, als vorläufige Addition $Add_{vorläufig}$ die mit dem unter Zuhilfenahme der Refraktionseinheit 10 nach der Figur 2 ermittelte Addition Add zu verwenden. Diese Refraktionseinheit 10 kann beispielsweise eine Sehprobentafel oder eine von innen beleuchtete Transparenttafel umfassen, wobei sich die Unterkante der Sehprobe mindestens 1,40m über dem Fußboden befinden muss. An der gegenüberliegenden Wand kann beispielsweise ein Umlenkspiegel angebracht sein, der so geneigt werden kann, dass der Prüfling das Bild der Sehprobe in der Mitte des Umlenkspiegels erkennt. Die Spiegelmitte liegt annähernd in Augenhöhe des sitzenden Prüflings. Weitere Details sind z.B. Heinz Diepes "Refraktionsbestimmung" Verlag Heinz Postenrieder, Pforzheim, 2. Auflage, 1975, S. 28 zu entnehmen.

In einem zweiten Schritt 200 wird die individuelle Schärfentiefe T des Auges des Probanden bestimmt. Die individuelle Schärfentiefe T des Auges des Probanden kann auf unterschiedlichste Art und Weise objektiv bestimmt werden. Eine Reihe von Verfahren zur objektiven Bestimmung der individuellen Schärfentiefe T wird im Weiteren beschrieben. Insbesondere kann auch eine Messeinheit 20 zu objektiven Bestimmung der Wellenfront und der Schärfentiefe T verwendet werden, wie Sie in Figur 2 dargestellt ist.

Die tatsächliche Addition Add ergibt sich dann in einem dritten Schritt 300 aus der vorläufigen Addition $Add_{vorläufig}$ abzüglich der mit einem im Bereich zwischen 0 und 1 liegenden Faktor $\omega$ gewichteten individuellen Schärfentiefe T. Als Gewichtungsfaktor $\omega$ kann z.B. ½ oder auch $2/3$ angesetzt werden. Die Berechnung kann z.B. mit einem herkömmlichen Personalcomputer 30 durchgeführt werden, wie er in Figur 2 skizziert ist. Additionsbestimmung, Schärfentiefebestimmung und Additionsberechnung kann auch in einem einzigen Computer erfolgen, der die Komponenten 10, 20 und 30 in sich vereinigt.

Die Bestimmung der Addition Add kann monokular oder auch binokular, sequentiell für die Sehhilfen (z.B. Brille) beider Augen getrennt durchgeführt werden. Es können Sehhilfen mit unterschiedlichen Additionen $Add_{links}$, $Add_{rechts}$ für das rechte und linke Auge des Probanden vorgesehen sein. Es ist jedoch auch möglich, dass z.B. für die Sehhilfen beider Augen des Probanden die gleiche Addition $Add = Add_{links} = Add_{rechts}$ verwendet wird. Als gemeinsame Addition Add für beide Sehhilfen (z.B. die Linsen einer Brille) kann z.B. die größte der für die jeweiligen Sehhilfen der Augen bestimmten Additionen $Add = \max[Add_{links}, Add_{rechts}]$ verwendet werden.

<u>Weiteres Ausführungsbeispiel zur Bestimmung der Addition</u>

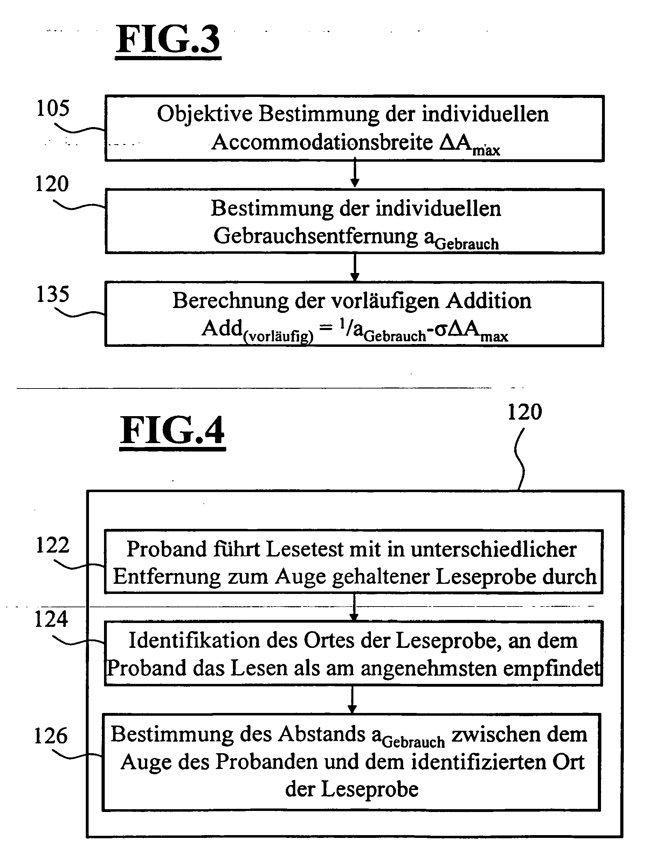

Die Figur 3 zeigt ein Flussdiagramm aus dem sich die einzelnen Verfahrensschritte einer weiteren Variante eines erfindungsgemäßen Verfahrens zur Bestimmung der individuell erforderlichen Addition Add einer Sehhilfe für einen Probanden entnehmen lassen.

In einem ersten Schritt 105 wird die individuelle Akkommodationsbreite $\Delta A_{max}$ abweichend vom Stand der Technik nicht subjektiv, d.h. unter Berücksichtigung des vom Probanden geäußerten Seheindrucks, sondern objektiv z.B. mit einer der nachstehend beschriebenen Methoden ermittelt.

In einem zweiten Schritt 120 wird die individuelle Gebrauchsentfernung $a_{Gebrauch}$ des Probanden ermittelt. Die Bestimmung der individuellen Gebrauchsentfernung $a_{Gebrauch}$ des Probanden kann z.B. die in der Figur 4 dargestellte Verfahrensschritte umfassen. Im Beispiel wird dem Probanden ein Schriftstück als Leseprobe überreicht. Der Proband wird angehalten, das Schriftstück in einer ihm angenehmen Entfernung zu halten und den Inhalt des Schriftstücks vorzulesen (Verfahrensschritt 122). Dabei wird die relative Position des Schriftstücks zum Augenhauptpunkt $H_A$ ermittelt (Verfahrensschritt 124). Dies kann z.B. durch Auswertung eines von einer Kamera aufgenommenen Bilds des lesenden Probanden im Profil erfolgen. Danach wird der Abstand $a_{Gebrauch}$ zwischen dem Hauptpunkt $H_A$ des Auges des Probanden und dem Ort der Leseprobe ermittelt (Verfahrensschritt 126).

Dieser Abstand $a_{Gebrauch}$ stellt die Gebrauchsentfernung $a_{Gebrauch}$ dar. Es ist auch denkbar, dass sich auf der Leseprobe ein Entfernungssensor befindet (z.B. als Bestandteil eines Displays), welcher den Abstand $a_{Gebrauch}$ zum Augenhauptpunkt $H_A$ unmittelbar misst.

In einem Schritt 135 wird die Addition Add der Sehhilfe z.B. mit Hilfe einer geeigneten Recheneinheit wie einem Computer errechnet als Differenz zwischen dem Kehrwert der Gebrauchsentfernung $a_{Gebrauch}$ und der mit einem Faktor $\sigma$ gewichteten individuellen Akkommodationsbreite $\Delta A_{max}$. Der Gewichtsfaktor $\sigma$ kann dabei Werte in einem Bereich größer als 0 und kleiner als 1 annehmen. Nach der oben erwähnten Theorie von Reiner kann der Gewichtsfaktor $\sigma$ z.B. einen Wert von ½ aufweisen. Schober schlägt z.B. einen Wert von $^2/_3$ vor.

Ausführungsbeispiel zur Bestimmung der Akkommodationsbreite und der Schärfentiefe

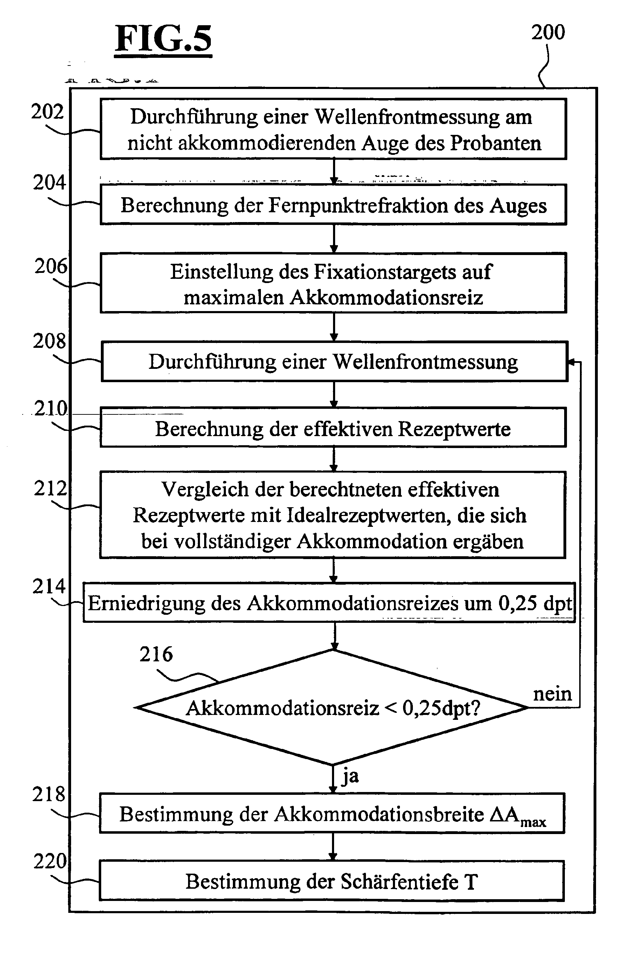

Ein Beispiel für ein Verfahren 200 zur Bestimmung der Akkommodationsbreite $\Delta A_{max}$ und der Schärfentiefe T, welches auf die Überlegungen der Erfinder zurück geht, umfasst die in der Figur 5 schematisch dargestellten und im Folgenden eingehend beschriebenen Verfahrensschritte:

Es erfolgt zunächst eine Messung 202 der an der Retina reflektierten Wellenfront eines auf das nicht akkommodierende Auge des Probanden treffenden Lichtstrahls mit vorbestimmter Wellenfront. Alternativ kann auch eine Autorefraktormessung durchgeführt werden. Aus der gemessenen Wellenfront oder der Autorefraktormessung wird dann die Fernpunktrefraktion $A_R$ des Auges berechnet (Verfahrensschritt 204).

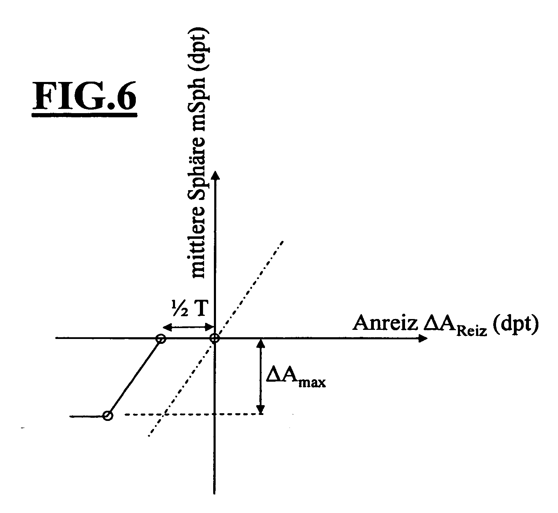

Erst dann wird die eigentliche Messung der Akkommodationsbreite $\Delta A_{max}$ durchgeführt. Dazu wird das Fixationstarget der Wellenfrontmesseinrichtung oder der Autorefraktormesseinrichtung an die Grenze des unteren Messbereiches gebracht (Schritt 206). Dies stellt den am Gerät maximal möglichen Akkommodationsreiz $\Delta A_{Reiz}$ für das Auge eines Probanden dar. Die Ideallinie zwischen Akkommodationsreiz $\Delta A_{Reiz}$ in dpt und Akkommodationserfolg $\Delta A$ in dpt ist in der Software festgelegt. Zur Veranschaulichung zeigt die Figur 6 den Zusammenhang zwischen der mittleren Sphäre mSph (entsprechend Gleichung (14)) des Auges eines Probanden und dem Akkommodationsreiz $\Delta A_{Reiz}$, d.h. dem Kehrwert des Abstands des Fixationstargets vom Augenhauptpunkt $H_A$. Die gestrichelte linear verlaufende Linie stellt den Idealfall dar, wenn die sich in einer entsprechenden mittleren Sphäre mSph widerspiegelnde Akkommodation A dem Akkommodationsreiz $\Delta A_{Reiz}$ stets folgt.

Nun erfolgen eine Messung der Wellenfront oder der Autorefraktorwerte (Verfahrensschritt 208) und daraus eine Berechnung der effektiven Rezeptwerte Sphäre, Zylinder, Achse des Auges (Verfahrensschritt 210). Diese effektiven Rezeptwerte Sphäre, Zylinder, Achse werden mit der Einstellung des Fixationstargets verglichen und gespeichert (Schritt 212). Nun wird das Fixationstarget in schnellen Schritten mit positiver sphärischer Wirkung Sph verändert (Schritt 212). Anders ausgedrückt bedeutet das, dass der Fixationsabstand vergleichsweise schnell verkleinert wird. Schnell bedeutet hier, dass das Auge der Änderung des Akkommodationsreizes $\Delta AR_{eiz}$ nicht folgen kann. Zu jeder Veränderung des Targets in Richtung zunehmender positiver sphärischer Wirkung Sph plus findet eine neue Wellenfrontmessung oder Autorefraktormessung mit anschließender Berechnung der Fernpunktrefraktion $A_R$ des Auges statt. Eine Veränderung des Targets mit positiver sphärischer Wirkung Sph findet bis Fernpunktrefraktion $A_R$ 3 dpt + 3dpt statt (Schritt 216). Die Akkommodationsbreite $\Delta A_{max}$ ergibt sich dann in der Graphik der Figur 6 aus der vertikalen Differenz zwischen dem Punkt auf der Messlinie, bei dem der Proband letztmalig nicht akkommodiert hat und dem Punkt bei dem der Proband erstmalig maximal akkommodiert hat (Schritt 218). Die Schärfentiefe T ergibt sich aus der zweifachen horizontalen Differenz zwischen dem Punkt auf der Messlinie, welche an der Fernpunktrefraktion $A_R$ liegt und dem Punkt an dem der Proband letztmalig nicht akkommodiert hat (Schritt 220, Figur 6).

Weiteres Ausführungsbeispiel zur Bestimmung der Akkommodationsbreite und der Schärfentiefe

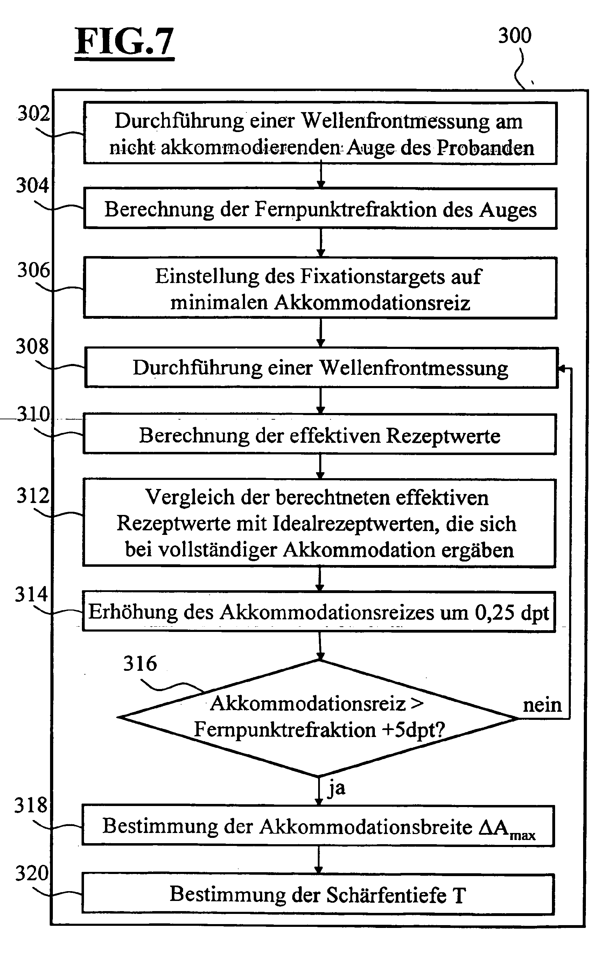

Ein weiteres Beispiel für ein Verfahren 300 zur Bestimmung der Akkommodationsbreite $\Delta A_{max}$ und der Schärfentiefe T umfasst die in der Figur 7 schematisch dargestellten und im Folgenden eingehend beschriebenen Verfahrensschritte:

Zunächst erfolgt wie beim vorangegangenen Beispiel eine Messung 302 der an der Retina reflektierten Wellenfront eines auf das nicht akkommodierende Auge des Probanden treffenden Lichtstrahls mit vorbestimmter Wellenfront. Alternativ kann selbstverständlich auch eine Autorefraktormessung durchgeführt werden. Aus der gemessenen Wellenfront oder der Autorefraktormessung wird dann die Fernpunktrefraktion $A_R$ des Auges berechnet (Verfahrensschritt 304).

Anschließend wird das Target der Wellenfrontmesseinrichtung oder der Autorefraktormesseinrichtung an die Grenze des oberen Messbereiches gebracht (Schritt 306).

Nun erfolgen eine Messung der Wellenfront (Schritt 308) oder der Autorefraktorwerte und daraus eine Berechnung der effektiven Rezeptwerte (sphärische Wirkung, zylindrische Wirkung, und Achslage) Sph, Zyl, Achse des Auges (Schritt 310). Diese Rezeptwerte werden mit der Einstellung des Targets verglichen (Schritt 312) und gespeichert. Nun wird das Target in schnellen Schritten in Richtung negativer sphärischer Wirkung Sph verändert. Zu jeder Veränderung des Targets in Richtung Minus findet eine neue Wellenfrontmessung oder Autorefraktormessung mit anschließender Berechnung der Fernpunktrefraktion $A_R$ des Auges statt (Schritt 316).

Die Akkommodationsbreite $\Delta A_{max}$ ergibt sich dann im Graph nach der Figur 6 aus der vertikalen Differenz zwischen dem Punkt auf der Messlinie, bei dem der Proband letztmalig maximal akkommodiert hat und dem Punkt bei dem der Proband erstmalig nicht akkommodiert hat (Schritt 318). Die Schärfentiefe T ergibt sich aus der zweifachen horizontalen Differenz zwischen dem Punkt auf der Messlinie, welche an der Fernpunktrefraktion liegt und dem Punkt an dem der Proband letztmalig nicht akkommodiert hat (Schritt 320, Figur 6).

Ausführungsbeispiel zur Bestimmung der Schärfentiefe

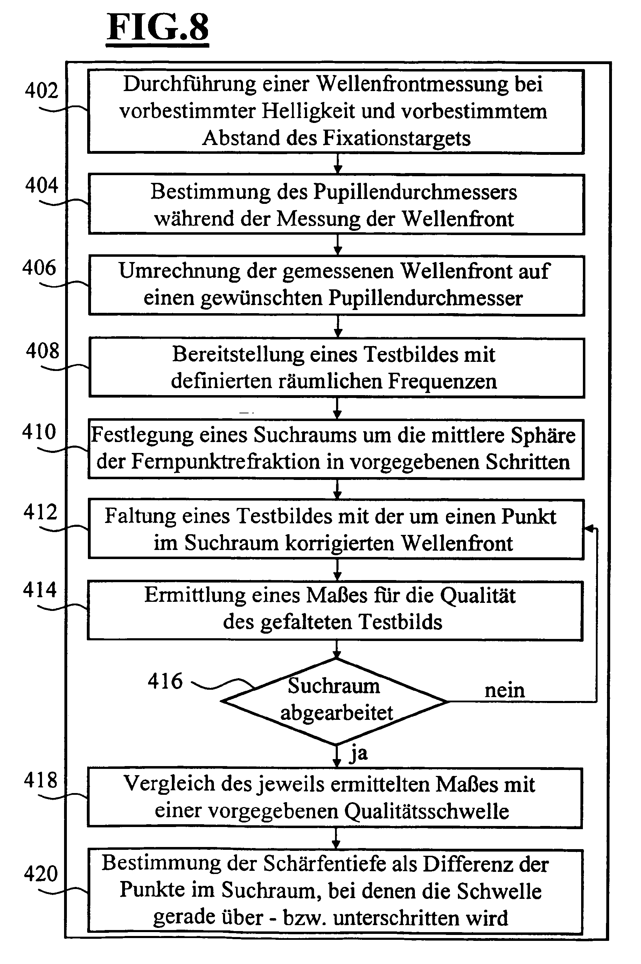

Ein Verfahren zur Bestimmung der Schärfentiefe T auf Basis einer Wellenfrontanalyse 400 kann auch nachfolgend angegebene und in Figur 8 skizzierte Verfahrensschritte umfassen:

In einem ersten Schritt wird bei vorbestimmten Helligkeitsverhältnissen und bei vorbestimmtem Abstand eines Fixationstargets eine Wellenfrontmessung an einem Auge des Probanden durchgeführt (Schritt 402). Die Messbedingungen ergeben sich aus der individuellen Problemstellung des Brillenträgers, also aus der Anamnese und können somit hier nicht eindeutig festgelegt werden.

Die vorbestimmte Helligkeit bestimmt die Pupillengröße des Auges des Probanden bei der Messung. Es ist bekannt, dass die sich aufgrund der Helligkeitsverhältnisse einstellende Pupillengröße die gemessene Wellenfront maßgeblich beeinflusst. Die Bedingungen bei der Messung der. Wellenfront sind jedoch nicht zwingend identisch mit den Bedingungen, unter denen der Proband üblicherweise einen Gegenstand betrachtet, insbesondere unter denen er z.B. Zeitung liest. Die Erfindung sieht daher in einem weiteren Schritt 406 die Umrechnung der gemessenen Wellenfront inkl. des gemessenen Pupillendurchmessers $P_{Messung}$ auf einen gewünschten Pupillendurchmesser $P_{Ziel}$ vor. Der gewünschte Pupillendurchmesser $P_{Ziel}$ kann z.B. der Pupillendurchmesser sein, der sich einstellen würde, wenn der Proband unter Tageslichtbedingungen Zeitung lesen würde. Der gewünschte Pupillendurchmesser $P_{Ziel}$ kann z.B. auch der Durchmesser sein, der sich einstellen würde, wenn der Proband unter künstlicher Beleuchtung am Computerbildschirm arbeiten würde. Es ist einleuchtend, dass sich die entsprechenden sich einstellenden Pupillendurchmesser $P_{Ziel}$ unter den meisten in Betracht kommenden Gebrauchsbedingungen objektiv z.B. mittels einer Videoaufnahme bestimmen lassen.

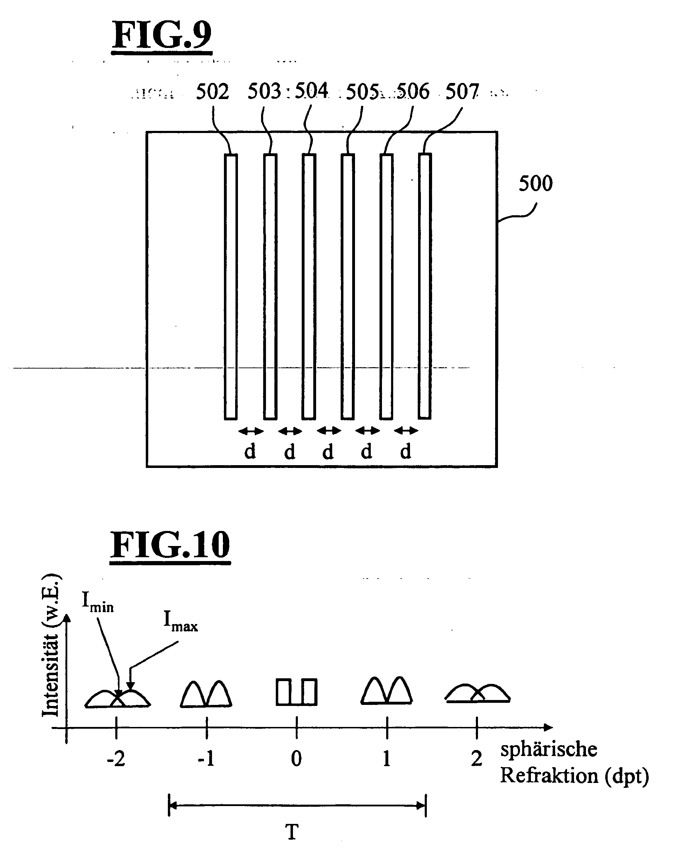

In einem weiteren Verfahrensschritt wird ein Fixationstarget mit definierten räumlichen Frequenzen entsprechend den gewünschten Anforderungen; z.B. Visus V = 0,4 beim Lesen festgelegt. Ein Beispiel für ein derartiges Muster (Testbild 500) zeigt die Figur 9. Das Muster besteht in diesem Beispiel aus sechs Streifen 502, 503, 504, 505, 506, 507, die in gleichem Abstand d zueinander angeordnet sind. Dieses Fixationstarget 500 wird dem Probanden jedoch nicht wie allgemein üblich zur Betrachtung in einem vorbestimmten Abstand $a_{Gebrauch}$ dargeboten, sondern es wird rechnerisch z.B. mit Hilfe eines Computers ermittelt, wie der Proband das Muster des Fixationstargets 500 wahrnehmen würde, wenn ihm dieses in verschiedenen Abständen $a_{Gebrauch}$ zum Augenhauptpunkt $H_A$ zur Ansicht bereitgestellt würde. Konkret erfolgt dies im Beispiel dadurch, dass zunächst ein Suchraum $R_s$ von z.B. +/-3 dpt oder +/-5 dpt um die mittlere Sphäre mSph der Fernpunktrefraktion $A_R$ in z.B. 0.05 dpt oder 0.1 dpt Schritten festgelegt wird (Schritt 410). Daraufhin erfolgt eine Faltung des Musters des Targets 500 mit der um den Punkt im Suchraum korrigierten Wellenfront (Verfahrensschritt 412), d.h. eine Bestimmung des aufgrund des veränderten Abstands $a_{Gebrauch}$ des Fixationstargets 500 zum Augenhauptpunkt $H_A$ unterschiedlich wahrnehmbaren Musters 502, 503, 504, 505, 506 und 507 des Fixationstargets 500.

In einem darauffolgenden Schritt 414 erfolgt eine Auswertung des durch die Faltung erhaltenen Musters 502, 503, 504, 505, 506 und 507 des Fixationstargets 500. Die Figur 10 zeigt die Intensitätsprofile zweier benachbarter Linien (z.B. 503 und 504 aus Fig. 9) des Musters 502, 503, 504, 505, 506 und 507 des Fixationstargets 500 nach der Faltung in Abhängigkeit von der mittleren Sphäre mSph, d.h. der Summe aus den gemessenen sphärischen und halben zylindrischen Refraktionswerten (Sph + Zyl/2), angegeben. Ein Maß für die Qualität der Wahrnehmbarkeit des Musters ist die getrennte Wahrnehmbarkeit benachbarter Linien mit erhöhter Intensität I, der photometrische Kontrast. Aus "Principles of Optics" (a.a.O.) ist es z.B. bekannt, dass Intensitätsunterschiede $\Delta I$ nebeneinander liegender Bereiche dann getrennt wahrnehmbar sind, wenn die geringere Intensität $I_{min}$ mehr als 19 % von der der stärkeren Intensität $I_{min}$

abweicht. Die Erfindung sieht daher vor, z.B. das Intensitätsmaximum $I_{max}$ und das Intensitätsminimum $I_{min}$ des ermittelten Intensitätsprofils I (wie z.B. die Figur 10 zeigt) zu bestimmen, die Differenz $\Delta I$ dieser ermittelten Extremwerte $I_{min}$, $I_{max}$ zu berechnen und den daraus erhaltenen Wert mit einem vorgegebenen Schwellwert S (wie z.B. dem Wert von 19 %) zu vergleichen und zwar für alle Werte $mSph_{korr}$ innerhalb des Suchraumes (Schritte 416, 418). Als Schärfentiefe T wird dann gerade die Differenz der Punkte im Suchraum festgelegt, bei denen die Schwelle S gerade über- bzw. unterschritten wird.

Weiteres Ausführungsbeispiel zur Bestimmung der Schärfentiefe

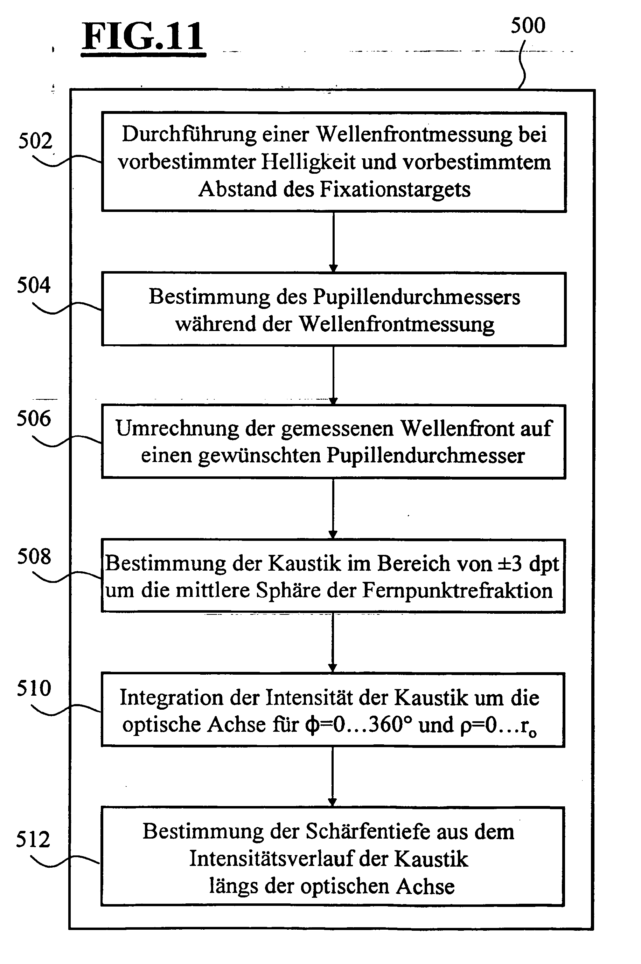

Ein weiteres Verfahren zur Bestimmung der Schärfentiefe T auf Basis einer Wellenfrontanalyse wird nachfolgend anhand der Figuren 11 bis 13 erläutert.

Das Verfahren geht wiederum von einer Wellenfrontmessung 502 bei vorbestimmten Helligkeitsverhältnissen und bei vorbestimmtem Abstand $a_{Fixation}$ eines Fixationstargets aus. Danach erfolgt wie im vorstehend beschriebenen Ausführungsbeispiel eine Umrechnung 504 der gemessenen Wellenfront auf einen gewünschten Pupillendurchmesser $PZ_{iel}$ (Schritt 506).

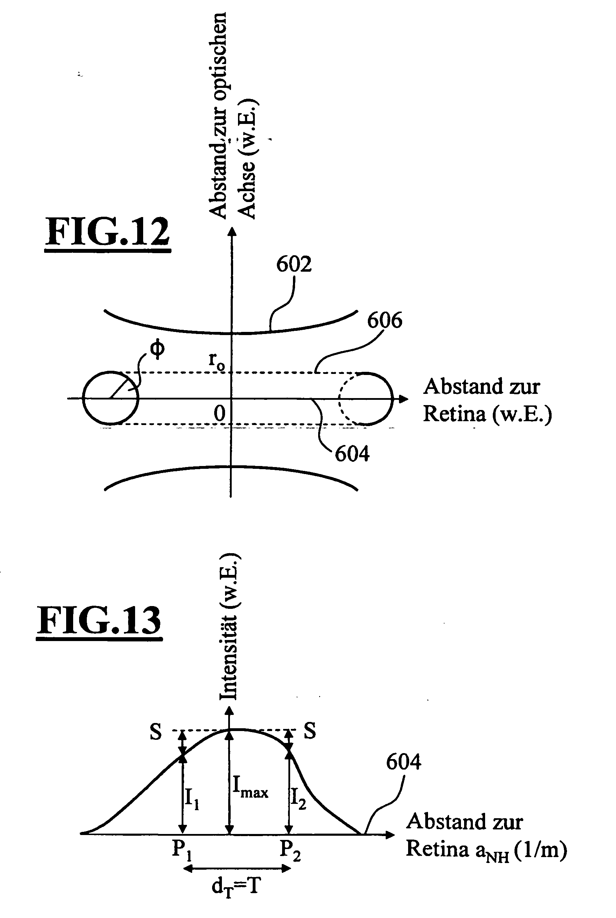

In einem weiteren Schritt 508 wird rechnerisch die Kaustik im Bereich von z.B. $\pm$ 3 dpt oder $\pm$ 5 dpt um die mittlere Sphäre der Fernpunktrefraktion $A_R$ bestimmt. Unter Kaustik 602 versteht man, wie dies in der Figur 12 skizziert ist, die mehr oder weniger enge Einschnürung eines Strahlenbündels, die anstelle eines Bildpunkts entsteht, welches das von einem Objektpunkt ausgehende Strahlenbündel infolge von Abbildungsfehlern zeigt, bevor es wieder auseinanderläuft. Konkret wird z.B. rechnerisch die Punktstreufunktion PSF an einer Mehrzahl an Orten $a_{NH}$ um die Retina des auf den Fernpunkt in Abstand $a_R$ gerichteten, nicht akkommodierenden Auges des Probanden ermittelt.

Danach erfolgt eine Integration 510 der Intensität der Kaustik 602 bis zu einem vorgegebenen Abstand $r_0$ zur optischen Achse 604. Diese Integration der Intensität der Kaustik 602 veranschaulicht das zylindrische Volumen 606 in der Figur 12, welches durch den Integrationswinkel $\varphi$ = 0 ... 360° und den Abstand p = 0 ... $r_0$ zur optischen Achse 604 gegeben ist. Der Abstand $r_0$ kann z.B. gleich dem Kehrwert des geforderten Visus V gesetzt werden. Die Figur 13 zeigt den innerhalb des zylindrischen Volumens 606 eingeschlossenen Intensitätsverlauf der Kaustik 602 längs der optischen Achse 604.

Schließlich wird die Schärfentiefe T der Darstellung der Figur 13 folgend aus dem Abstand $d_T$ der beiden Punkte $P_1$, $P_2$ entlang der optischen Achse 604 bestimmt, an denen die Intensität $I_1$, $I_2$ um einen bestimmten Schwellwert S, z.B. 19%, kleiner als die maximale Intensität $I_{max}$ ist (Schritt 512).

Ausführungsbeispiel zur Bestimmung der Schärfentiefe für eine andere Gebrauchsbedingung

Es hat sich gezeigt, dass die Schwelle S, ab der unterschiedliche Intensitäten $I_i$ als solche wahrgenommen werden können stark vom jeweiligen Probanden abhängt. Es ist daher sinnvoll, diese Schwelle S individuell zu ermitteln und damit die Schärfentiefe T noch präziser zu bestimmen.

Ein Verfahren zur individuellen Bestimmung der Schärfentiefe T auf Basis einer Wellenfrontanalyse skaliert auf einen gewünschten oder vorgegebenen Pupillendurchmesser $P_{Ziel}$ kann folgende Verfahrensschritte umfassen:

Aus einem der oben zuerst beschriebenen Verfahren erhält man z.B. die Schärfentiefe T1 für einen ersten Pupillendurchmesser P1. Nach einem der alternativen o.a. Verfahren wird mit einem zweiten Pupillendurchmesser P2 = P1 die individuelle Schwelle $S_{ind}$ so bestimmt, dass die daraus berechnete Schärfentiefe T2 gleich der gemessenen Schärfentiefe T1 ist.

Daraufhin kann mit einem der alternativen o.a. Verfahren für einen vorgegebenen Pupillendurchmesser $P_{Ziel}$ (z.B. 3,5mm) und der im vorigen Schritt bestimmten Schwelle $S_{ind}$ die Schärfentiefe T bestimmt werden. Falls P1 kleiner als der gewünschte Pupillendurchmesser $P_{Ziel}$ ist, wird T2=T1 gesetzt.

Resümee

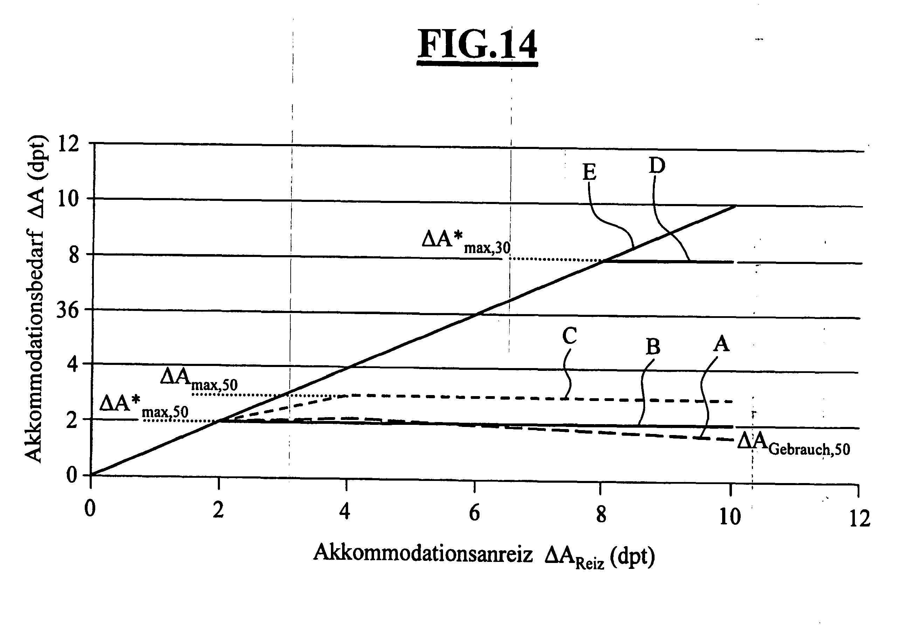

Die Figur 14 zeigt zur Verdeutlichung der Unterschiede zum Stand der Technik ein Diagramm, in dem die Akkommodation A eines Auges unterschiedlicher Probanden über dem Akkommodationsanreiz $\Delta A_{Reiz}$ aufgetragen ist. Kurve E stellt den linearen Verlauf der idealen Akkommodation $A_{ideal}$ dar. Das ideale Auge ist in der Lage jeden Akkommodationsanreiz $\Delta A_{max}$ durch eine entsprechende Akkommodation $A_{ideal}$ auszugleichen.

Zum Vergleich ergibt sich aus der Kurve D die mittlere Akkommodation $A_{m,\,30}$ eines 30 Jährigen und aus Kurve C die mittlere Akkommodation $A_{m,\,50}$ eines 50 Jährigen nach Duane. Das Auge eines durchschnittlichen Dreißigjährigen ist in der Lage einen Akkommodationsanreiz $\Delta A_{Reiz}$ bis zu 8 dpt auszugleichen. Für höhere Akkommodationsanreize $\Delta A_{Reiz}$ fehlt die Elastizität der Linse seines Auges. Das Auge eines durchschnittlichen Fünfzigjährigen ist nur noch in der Lage einen Akkommodationsanreiz $\Delta A_{Reiz}$ bis zu 2 dpt auszugleichen, weil die Elastizität der Linse seines Auges bereits derart abgenommen hat. Seine Restakkommodation $\Delta A_{max}$ beträgt 2 dpt.

Es ist dem Fachmann einleuchtend, dass die Akkommodationsbreite $\Delta A_{max}$ individuell sehr unterschiedlich ist und nicht allein vom Alter abhängt. Die Kurve A stellt die individuell ermittelte Gebrauchsakkommodation $\Delta A_{Gebrauch}$ einer 50-jährigen Versuchperson dar. Die Kurve B ist die zugehörige individuelle Akkommodation $A_{50}$ des 50-jährigen Probanden. Es zeigt sich, dass das Auge des Probanden tatsächlich noch eine Akkommodationsbreite $\Delta A_{max}$ von 3 dpt besitzt. Genutzt wird eine Gebrauchsakkommodation $\Delta A_{Gebrauch}$ von etwa 2 dpt.

Das erfindungsgemäße Verfahren und die erfindungsgemäße Vorrichtung berücksichtigt die objektiv bestimmbaren und ggf. auch objektiv bestimmten Parameter Akkommodationsbreite $\Delta A_{max}$ und Schärfentiefe T. Das bedeutet, dass das neue Verfahren nicht auf die Kompilanz des Probanden angewiesen ist. Dies ist insbesondere von Vorteil, wenn Sehhilfen für ältere oder kranke Personen angepasst werden sollen. Darüber hinaus ist es nicht erforderlich, auf die übliche 0,25 dpt Quantisierung der Addition Add zurückzugreifen, sondern die Addition Add kann individuell angepasst sein, was einen weiteren Schritt zur individuell optimierten Sehhilfe bedeutet. Die Berücksichtigung der individuellen Schärfentiefe T hat den Vorteil, dass die Addition Add so klein wie unbedingt erforderlich gewählt werden kann, mit dem Ergebnis, dass insbesondere Brillenlinsen mit weichem Design hergestellt werden können, ohne Sehschärfe im Nahbereich zu verlieren.

**Patentansprüche**

1. Verfahren zur Bestimmung der individuell erforderlichen Addition (Add) einer Sehhilfe für ein Auge mit Hilfe eines Computers, mit folgenden Verfahrensschritten:

   a) eine vorläufige Addition (Add$_{vorläufig}$) wird bestimmt,
   b) die Schärfentiefe (T) des Auges wird individuell bestimmt,
   c) die Addition (Add) wird nach folgender Gleichung berechnet:

   $$Add = Add_{vorläufig} - \omega T$$, wobei $\omega$ eine reelle Zahl darstellt, die im Bereich $0 < \omega \leq 1$ liegt.

2. Verfahren nach Anspruch 1, **dadurch gekennzeichnet, dass**

   a) der maximale Akkommodationsreiz ($\Delta A_{Reiz, max}$) bestimmt wird, an dem das Auge gerade noch nicht akkommodiert, und dass
   b) die Schärfentiefe (T) dem zweifachen des maximalen Akkommodationsreizes ($\Delta A_{Reiz,max}$) gleichgesetzt wird.

3. Verfahren zur Bestimmung der individuell erforderlichen Addition (Add) einer Sehhilfe für ein Auge mit Hilfe eines Computers, mit folgenden Verfahrensschritten:

   a) die Akkommodationsbreite ($\Delta A_{max}$) wird individuell und objektiv bestimmt,
   b) die Gebrauchsentfernung (a$_{Gebrauch}$) wird individuell bestimmt,
   c) die Addition (Add) wird nach folgender Gleichung berechnet:

   $$Add = 1/a_{Gebrauch} - \sigma \Delta A_{max}$$, wobei $\sigma$ eine reelle Zahl darstellt, die im Bereich $0 < \sigma \leq 1$ liegt.

4. Verfahren nach Anspruch 3, **dadurch gekennzeichnet, dass** die Gebrauchsentfernung (a$_{Gebrauch}$) durch Anamnese mit Angabe der Hauptsehaufgaben und den dazugehörigen Entfernungen durch den Probanden bestimmt wird.

5. Verfahren nach einem der Ansprüche 3 oder 4, **dadurch gekennzeichnet, dass** die tatsächliche Akkommodationsbreite ($\Delta A_{max}$) wie folgt bestimmt wird:

   a) die Fernpunktrefraktion (A$_R$) und die Nahpunktrefraktion (A$_p$) wird aus einer Wellenfrontmessung oder einer Autorefraktionsmessung des Auges bestimmt,
   b) die tatsächliche Akkommodationsbreite ($\Delta A_{max}$) wird als Differenz zwischen der in Schritt i) gemessenen Fernpunktrefraktion (A$_R$ und der in Schritt a) gemessenen Nahpunktrefraktion (A$_p$) berechnet.

6. Verfahren nach einem der Ansprüche 1 oder 2, **dadurch gekennzeichnet, dass** die vorläufige Addition (Add$_{vorläufig}$) nach folgender Gleichung berechnet wird:

   $$Add_{vorläufig} = A_p$$, wobei $A_p$ die Nahpunktrefraktion darstellt.

7. Vorrichtung zur Bestimmung der individuell erforderlichen Addition (Add) einer Sehhilfe für ein Auge mit:

a) einer Additionsbestimmungseinrichtung zur Bestimmung einer vorläufigen Addition (Add$_{vorläufig}$),
b) einer Schärfentiefebestimmungseinrichtung, um die Schärfentiefe (T) des Auges individuell zu bestimmen,
c) einer Additionsberechnungseinrichtung, um die Addition (Add) nach folgender Gleichung zu berechnen:

$$Add = Add_{vorläufig} - \omega T$$, wobei $\omega$ eine reelle Zahl darstellt, die im Bereich $0 < \omega \le 1$ liegt.

8. Vorrichtung nach Anspruch 7, **dadurch gekennzeichnet, dass**

i) eine Akkommodationsreizbestimmungseinrichtung vorgesehen ist, um den maximalen Akkommodationsreiz ($\Delta A_{Reiz,\ max}$) zu bestimmen, an dem das Auge gerade noch nicht akkommodiert, und dass
ii) die Schärfentiefebestimmungseinrichtung eingerichtet ist, die Schärfentiefe (T) mit dem zweifachen des maximalen Akkommodationsreizes ($\Delta A_{Reiz,\ max}$) gleichzusetzen.

9. Vorrichtung nach Anspruch 8, **dadurch gekennzeichnet, dass** die Akkommodationsreizbestimmungseinrichtung eine Wellenfrontmesseinrichtung oder einer Autorefraktionsmesseinrichtung umfasst.

10. Vorrichtung nach einem der Ansprüche 7 bis 9, **dadurch gekennzeichnet, dass** die Additionsbestimmungseinrichtung eingerichtet ist, um eine individuelle Messung der physiologischen Akkommodationsbreite ($\Delta A^{*}_{max}$) und/oder eine individuelle Messung der tatsächlichen Akkommodationsbreite ($\Delta A_{max}$) und/oder eine individuelle Messung der tatsächlichen Gebrauchsakkommodation ($\Delta A_{Gebrauch}$) zu bestimmen.

11. Vorrichtung nach Anspruch 10, **dadurch gekennzeichnet, dass** eine Gebrauchsentfernungsbestimmungseinrichtung zur Bestimmung der Gebrauchsentfernung ($a_{Gebrauch}$) vorgesehen ist und dass die Additionsbestimmungseinrichtung eingerichtet ist, die vorläufige Addition (Add$_{vorläufig}$) nach folgender Gleichung zu berechnen:

$$Add_{vorläufig} = 1/a_{Gebrauch} - \sigma \Delta A^{*}_{max}$$, wobei $\sigma$ eine reelle Zahl darstellt, die im Bereich $0 \le \sigma \le 1$ liegt.

12. Vorrichtung nach Anspruch 10, **dadurch gekennzeichnet, dass** eine Gebrauchsentfernungsbestimmungseinrichtung zur Bestimmung der Gebrauchsentfernung ($a_{Gebrauch}$) vorgesehen ist und dass die Additionsbestimmungseinrichtung eingerichtet ist, die vorläufige Addition (Add$_{vorläufig}$) nach folgender Gleichung zu berechnen:

$$Add_{vorläufig} = 1/a_{Gebrauch} - \sigma \Delta A_{max}$$, wobei $\sigma$ eine reelle Zahl darstellt, die im Bereich $0 \le \sigma \le 1$ liegt.

13. Vorrichtung zur Bestimmung der individuell erforderlichen Addition (Add) einer Sehhilfe für ein Auge mit:

a) einer Akkommodationsbreitenbestimmungseinrichtung zur individuellen und objektiven Bestimmung der Akkommodationsbreite ($\Delta A_{max}$),
b) einer Gebrauchsentfernungsbestimmungseinrichtung zur individuellen Bestimmung der Gebrauchsentfernung ($a_{Gebrauch}$),
c) einer Additionsberechnungseinrichtung zur Berechnung der Addition (Add) nach folgender Gleichung:

$$Add = 1/a_{Gebrauch} - \sigma \Delta A_{max}$$, wobei $\sigma$ eine reelle Zahl darstellt, die im Bereich $0 < \sigma \le 1$ liegt.

14. Computerprogramm mit Programmcode eingerichtet zur Ausführung des Verfahrens nach einem der Ansprüche 1 und 2, wenn das Programm auf einer Vorrichtung gemäss Anspruch 7 geladen wird.

15. Computerprogramm mit Programmcode eingerichtet zur Ausführung des Verfahrens nach einem der Ansprüche 3 - 6, wenn das Programm auf einer Vorrichtung gemäss Anspruch 13 geladen wird.

**Claims**

1. Method for determining the individually required addition (Add) of a visual aid for an eye with the aid of a computer, comprising the following method steps:

a) determining a preliminary addition (Add$_{preliminary}$),

b) individually determining the depth-of-field (T) of the eye,
c) calculating the addition (Add) according to the following equation:

$$Add = Add_{preliminary} - \omega T, \text{ with } \omega \text{ being a real number in the range } 0 < \omega \leq 1.$$

2. Method according to Claim 1, **characterized in that**

a) the maximum accommodation stimulus ($\Delta A_{stimulus,max}$) at which the eye does not yet quite accommodate is determined, and **in that**
b) the depth-of-field (T) is equated to twice the maximum accommodation stimulus ($\Delta A_{stimulus,max}$).

3. Method for determining the individually required addition (Add) of a visual aid for an eye with the aid of a computer, comprising the following method steps:

a) individually and objectively determining the accommodation range ($\Delta A_{max}$),
b) individually determining the usage distance ($a_{usage}$,
c) calculating the addition (Add) according to the following equation:

$$Add = 1/a_{usage} - \sigma \Delta A_{max}, \text{ with } \sigma \text{ being a real number in the range } 0 < \sigma \leq 1.$$

4. Method according to Claim 3, **characterized in that** the usage distance ($a_{usage}$) is determined by the medical history with specifications by the subject of the main viewing tasks and the associated distances.

5. Method according to either of Claims 3 and 4, **characterized in that** the actual accommodation range ($\Delta A_{max}$) is determined as follows:

a) the far-point refraction ($A_R$) and the near-point refraction (Ap) is determined from a wavefront measurement or an autorefraction measurement of the eye,
b) the actual accommodation range ($\Delta A_{max}$) is calculated as the difference between the far-point refraction ($A_R$) measured in step a) and the near-point refraction ($A_p$) measured in step a).

6. Method according to either of Claims 1 and 2, **characterized in that** the preliminary addition ($Add_{preliminary}$) is calculated according to the following equation:

$$Add_{preliminary} = A_p, \text{ with } A_p \text{ being the near-point refraction.}$$

7. Device for determining the individually required addition (Add) of a visual aid for an eye, comprising:

a) an addition determination apparatus for determining a preliminary addition ($Add_{preliminary}$),
b) a depth-of-field determination apparatus for individually determining the depth-of-field (T) of the eye,
c) an addition calculation apparatus for calculating the addition (Add) according to the following equation:

$$Add = Add_{preliminary}y - \omega T, \text{ with } \omega \text{ being a real number in the range } 0 < \omega \leq 1.$$

8. Device according to Claim 7, **characterized in that**

i) provision is made for an accommodation stimulus determination apparatus for determining the maximum accommodation stimulus ($\Delta A_{stimulus,max}$) at which the eye does not yet quite accommodate, and **in that**
ii) the depth-of-field determination apparatus is designed to equate the depth-of-field (T) to twice the maximum accommodation stimulus ($\Delta A_{stimulus,max}$).

9. Device according to Claim 8, **characterized in that** the accommodation stimulus determination apparatus comprises a wavefront measurement apparatus or an autorefraction measurement apparatus.

10. Device according to one of Claims 7 to 9, **characterized in that** the addition determination apparatus is designed to determine an individual measurement of the physiological accommodation range ($\Delta A^*_{max}$) and/or an individual measurement of the actual accommodation range ($\Delta A_{max}$) and/or an individual measurement of the actual usage accommodation ($\Delta A_{usage}$).

**11.** Device according to Claim 10, **characterized in that** provision is made for a usage distance determination apparatus for determining the usage distance ($a_{usage}$) and **in that** the addition determination apparatus is designed to calculate the preliminary addition ($Add_{preliminary}$) according to the following equation:

$$Add_{preliminary} = 1/a_{usage} - \sigma\Delta A^*_{max}, \text{ with } \sigma \text{ being a real number in the range } 0 < \sigma \leq 1.$$

**12.** Device according to Claim 10, **characterized in that** provision is made for a usage distance determination apparatus for determining the usage distance ($a_{usage}$) and **in that** the addition determination apparatus is designed to calculate the preliminary addition ($Add_{preliminary}$) according to the following equation:

$$Add_{preliminary} = 1/a_{usage} - \sigma\Delta A_{max}, \text{ with } \sigma \text{ being a real number in the range } 0 < \sigma \leq 1.$$

**13.** Device for determining the individually required addition (Add) of a visual aid for an eye, comprising:

a) an accommodation range determination apparatus for individually and objectively determining the accommodation range ($\Delta A_{max}$),
b) a usage distance determination apparatus for individually determining the usage distance usage),
c) an addition calculation apparatus for calculating the addition (Add) according to the following equation:

$$Add = 1/a_{usage} - \sigma\Delta A_{max}, \text{ with } \sigma \text{ being a real number in the range } 0 < \sigma \leq 1.$$

**14.** Computer program with program code designed to execute the method according to either of Claims 1 and 2 when the program is loaded on a device according to Claim 7.

**15.** Computer program with program code designed to execute the method according to one of Claims 3-6 when the program is loaded on a device according to Claim 13.

**Revendications**

**1.** Procédé de détermination de l'ajout individuel nécessaire (Add) d'une aide à la vision destiné à un oeil à l'aide d'un ordinateur, comportant les étapes de procédé suivantes :

a) un ajout provisoire ($Add_{provisoire}$) est déterminé,
b) la profondeur de champ (T) de l'oeil est déterminée individuellement ;
c) l'ajout (Add) est calculé conformément à l'équation suivante :

$$Add = Add_{provisoire} - \omega T, \text{ où } \omega \text{ est un nombre réel se situant dans l'intervalle } 0 < \omega \leq 1.$$

**2.** Procédé selon la revendication 1, **caractérisé en ce que**

a) le stimulus d'accommodation maximal ($\Delta A_{stimulus, max}$) pour lequel l'oeil n'est pas encore parfaitement accommodé est déterminé, et **en ce que**
b) la profondeur de champ (T) du stimulus d'accommodation est rendue égale au double du stimulus d'accommodation maximal ($\Delta A_{stimulus, max}$).

**3.** Procédé de détermination de l'ajout nécessaire individuel (Add) d'une aide à la vision destinée à un oeil à l'aide d'un ordinateur, comprenant les étapes de procédé suivantes :

a) l'amplitude d'accommodation ($\Delta A_{max}$) est déterminée individuellement et objectivement,
b) la distance utile ($a_{utile}$) est déterminée individuellement,
c) l'ajout (Add) est calculé conformément à l'équation suivante :

$$Add = 1/a_{utile} - \sigma\Delta A_{max}, \text{ où } \sigma \text{ est un nombre réel se situant dans l'intervalle } 0 < \sigma \leq 1.$$

**4.** Procédé selon la revendication 3, **caractérisé en ce que** la distance utile ($a_{utile}$) est déterminée par les sujets à partir des antécédents médicaux avec indication des fonctions de vision principales et des distances correspondantes.

**5.** Procédé selon l'une quelconque des revendications 3 ou 4, **caractérisé en ce que** l'amplitude d'accommodation réelle ($\Delta A_{max}$) est déterminée comme suit :

   a) la réfraction au punctum remotum ($A_R$) et la réfraction au punctum proximum ($A_p$) sont déterminées à partir d'une mesure du front d'onde ou d'une mesure d'auto-réfraction de l'oeil,
   b) l'amplitude d'accommodation réelle ($\Delta A_{max}$) est déterminée sous la forme de la différence entre la réfraction au punctum remotum ($A_R$) mesurée à l'étape a) et la réfraction au punctum proximum ($A_p$) mesurée à l'étape a).

**6.** Procédé selon l'une quelconque des revendications 1 ou 2, **caractérisé en ce que** l'ajout provisoire ($Add_{provisoire}$) est calculé conformément à l'équation suivante :

   $Add_{provisoire} = A_p$, où $A_p$ représente la réfraction au punctum proximum.

**7.** Procédé de détermination de l'ajout nécessaire individuel (Add) d'une aide à la vision destinée à un oeil, comprenant :

   a) un dispositif de détermination d'ajout destiné à déterminer un ajout provisoire ($Add_{provisoire}$),
   b) un dispositif de détermination de profondeur de champ destiné à déterminer individuellement la profondeur de champ (T) de l'oeil ;
   c) un dispositif de calcul d'ajout destiné à calculer l'ajout (Add) conformément à l'équation suivante :

   $Add = Add_{provisoire} - \omega T$, où $\omega$ est un nombre réel se situant dans l'intervalle $0 < \omega \leq 1$.

**8.** Dispositif selon la revendication 7, **caractérisé en ce que**

   i) il est prévu un dispositif de détermination de stimulus d'accommodation destiné à déterminer le stimulus d'accommodation maximal ($\Delta A_{stimulus,\ max}$) pour lequel l'oeil n'est pas accommodé de manière appropriée, et **en ce que**
   ii) le dispositif de détermination de profondeur de champ est conçu pour rendre égale la profondeur de champ (T) au double du stimulus d'accommodation maximal ($\Delta A_{stimulus,\ max}$).

**9.** Dispositif selon la revendication 8, **caractérisé en ce que** le dispositif de détermination de stimulus d'accommodation comprend un dispositif de mesure de front d'onde ou un dispositif de mesure d'auto-réfraction.

**10.** Dispositif selon l'une quelconque des revendications 7 à 9, **caractérisé en ce que** le dispositif de détermination d'ajout est conçu pour déterminer une mesure individuelle de l'amplitude d'accommodation physiologique ($\Delta A^*_{max}$) et/ou une mesure individuelle de l'amplitude d'accommodation réelle ($\Delta A_{max}$) et/ou une mesure individuelle de l'accommodation utile réelle ($\Delta A_{utile}$).

**11.** Dispositif selon la revendication 10, **caractérisé en ce qu'**il est prévu un dispositif de détermination de distance utile destiné à déterminer la distance utile ($a_{utile}$) et **en ce que** le dispositif de détermination d'ajout est conçu pour calculer l'ajout provisoire ($Add_{provisoire}$) conformément à l'équation suivante :

   $Add = 1/a_{utile} - \sigma \Delta A^*_{max}$, où $\sigma$ est un nombre réel se situant dans l'intervalle $0 < \sigma \leq 1$.

**12.** Dispositif selon la revendication 10, **caractérisé en ce qu'**il est prévu un dispositif de détermination de distance utile destiné à déterminer la distance utile ($a_{utile}$) et **en ce que** le dispositif de détermination d'ajout est conçu pour calculer l'ajout provisoire ($Add_{provisoire}$) conformément à l'équation suivante :

   $Add_{provisoire} = 1/a_{utile} - \sigma \Delta A_{max}$, où $\sigma$ est un nombre réel se situant dans l'intervalle $0 < \sigma \leq 1$.

**13.** Dispositif de détermination de l'ajout individuel nécessaire (Add) d'une aide à la vision destinée à un oeil, comprenant :

   a) un dispositif de détermination d'amplitude d'accommodation destiné à déterminer individuellement et objectivement l'amplitude d'accommodation ($\Delta A_{max}$),
   b) un dispositif de détermination de distance utile destiné à déterminer la distance utile ($a_{utile}$),
   c) un dispositif de calcul d'ajout destiné à calculer l'ajout (Add) conformément à l'équation suivante :

   $Add = 1/a_{utile} - \sigma \Delta A_{max}$, où $\sigma$ est un nombre réel se situant dans l'intervalle $0 < \sigma \leq 1$.

**14.** Programme informatique comprenant un code de programme conçu pour mettre en oeuvre le procédé selon l'une quelconque des revendications 1 et 2 lorsque le programme est chargé sur un dispositif selon la revendication 7.

**15.** Programme informatique comprenant un code de programme conçu pour mettre en oeuvre le procédé selon l'une quelconque des revendications 3 à 6 lorsque le programme est chargé sur un dispositif selon la revendication 13.

# FIG.1

100  | Bestimmung einer vorläufigen Addition Add$_{vorläufig}$

200  | Bestimmung der individuellen Schärfentiefe T

300  | Berechnung der Addition Add = Add$_{vorläufig}$ - ωT

# FIG.2

10

20

30

# FIG.3

105 — Objektive Bestimmung der individuellen Accommodationsbreite $\Delta A_{max}$

120 — Bestimmung der individuellen Gebrauchsentfernung $a_{Gebrauch}$

135 — Berechnung der vorläufigen Addition $Add_{(vorläufig)} = {}^1/a_{Gebrauch} - \sigma \Delta A_{max}$

# FIG.4

120

122 — Proband führt Lesetest mit in unterschiedlicher Entfernung zum Auge gehaltener Leseprobe durch

124 — Identifikation des Ortes der Leseprobe, an dem Proband das Lesen als am angenehmsten empfindet

126 — Bestimmung des Abstands $a_{Gebrauch}$ zwischen dem Auge des Probanden und dem identifizierten Ort der Leseprobe

# FIG.5

200

| Durchführung einer Wellenfrontmessung am nicht akkommodierenden Auge des Probanten |
| --- |

| Berechnung der Fernpunktrefraktion des Auges |
| --- |

| Einstellung des Fixationstargets auf maximalen Akkommodationsreiz |
| --- |

| Durchführung einer Wellenfrontmessung |
| --- |

| Berechnung der effektiven Rezeptwerte |
| --- |

| Vergleich der berechtneten effektiven Rezeptwerte mit Idealrezeptwerten, die sich bei vollständiger Akkommodation ergäben |
| --- |

| Erniedrigung des Akkommodationsreizes um 0,25 dpt |
| --- |

Akkommodationsreiz < 0,25dpt?   nein

ja

| Bestimmung der Akkommodationsbreite $\Delta A_{max}$ |
| --- |

| Bestimmung der Schärfentiefe T |
| --- |

FIG.6

# FIG.7

300

**302** Durchführung einer Wellenfrontmessung am nicht akkommodierenden Auge des Probanden

**304** Berechnung der Fernpunktrefraktion des Auges

**306** Einstellung des Fixationstargets auf minimalen Akkommodationsreiz

**308** Durchführung einer Wellenfrontmessung

**310** Berechnung der effektiven Rezeptwerte

**312** Vergleich der berechtneten effektiven Rezeptwerte mit Idealrezeptwerten, die sich bei vollständiger Akkommodation ergäben

**314** Erhöhung des Akkommodationsreizes um 0,25 dpt

**316** Akkommodationsreiz > Fernpunktrefraktion +5dpt?   nein

ja

**318** Bestimmung der Akkommodationsbreite $\Delta A_{max}$

**320** Bestimmung der Schärfentiefe T

# FIG.8

| | |
|---|---|
| 402 | Durchführung einer Wellenfrontmessung bei vorbestimmter Helligkeit und vorbestimmtem Abstand des Fixationstargets |
| 404 | Bestimmung des Pupillendurchmessers während der Messung der Wellenfront |
| 406 | Umrechnung der gemessenen Wellenfront auf einen gewünschten Pupillendurchmesser |
| 408 | Bereitstellung eines Testbildes mit definierten räumlichen Frequenzen |
| 410 | Festlegung eines Suchraums um die mittlere Sphäre der Fernpunktrefraktion in vorgegebenen Schritten |
| 412 | Faltung eines Testbildes mit der um einen Punkt im Suchraum korrigierten Wellenfront |
| 414 | Ermittlung eines Maßes für die Qualität des gefalteten Testbilds |

416 — Suchraum abgearbeitet — nein

ja

| | |
|---|---|
| 418 | Vergleich des jeweils ermittelten Maßes mit einer vorgegebenen Qualitätsschwelle |
| 420 | Bestimmung der Schärfentiefe als Differenz der Punkte im Suchraum, bei denen die Schwelle gerade über - bzw. unterschritten wird |

# FIG.9

502  503 : 504  : 505 : 506  507

500

d   d   d   d   d

# FIG.10

Intensität (w.E.)

$I_{min}$

$I_{max}$

-2   -1   0   1   2   sphärische
Refraktion (dpt)

T

# FIG.11

500

502 — Durchführung einer Wellenfrontmessung bei vorbestimmter Helligkeit und vorbestimmtem Abstand des Fixationstargets

504 — Bestimmung des Pupillendurchmessers während der Wellenfrontmessung

506 — Umrechnung der gemessenen Wellenfront auf einen gewünschten Pupillendurchmesser

508 — Bestimmung der Kaustik im Bereich von ±3 dpt um die mittlere Sphäre der Fernpunktrefraktion

510 — Integration der Intensität der Kaustik um die optische Achse für $\phi=0...360°$ und $\rho=0...r_o$

512 — Bestimmung der Schärfentiefe aus dem Intensitätsverlauf der Kaustik längs der optischen Achse

**FIG.12**

**FIG.13**

# FIG.14

EP 2 208 457 B1

FIG.15    Stand der Technik

physiologische Akkomodationsbreite $\Delta A^{*}_{max} = \Delta A_{max} + T$ (dpt)

Alter (Jahre)

802
804
806

EP 2 208 457 B1

**FIG.16**  Stand der Technik

minimale Sehweite $a_p$ (cm)

Alter (Jahre)

# FIG.17 Stand der Technik

**IN DER BESCHREIBUNG AUFGEFÜHRTE DOKUMENTE**

**In der Beschreibung aufgeführte Patentdokumente**

- US 6554429 B1
- WO 2008064379 A1

**In der Beschreibung aufgeführte Nicht-Patentliteratur**

- **HELMUT GOERSCH.** Wörterbuch der Optometrie. 2001
- **M. MILLODOT et al.** Presbyopia correction and the accommodation in reserve. *Ophthalmic & Physiological Optics UK,* April 1989, vol. 9 (2), 126-132
- Clinical predictors of the optimal spectacle correction for comfort performing desktop tasks. *Clinical and Experimental Optometry: Journal of the Australian Optometrical Association,* November 2008, vol. 91 (6), 530-537
- Subjective and objective measurement of human accomodative amplitude. *Journal of Cataract and Refractive Surgery,* Oktober 2003, vol. 29 (10), 1879-1888
- **HEINZ DIEPES.** Refraktionsbestimmung. Verlag Heinz Postenrieder, 1975, 414
- Scaling Zernike expansion coefficients to different pupil sizes. *J. Opt. Soc. Am. A,* vol. 19 (10), 1937-1945
- **MAX BORN et al.** Principles of Optics. Cambridge University Press, 370-371
- **HELMUT GOERSCH.** Zeiss Handbuch für Augenoptik. 2000, 35
- **HEINZ DIEPES.** Refraktionsbestimmung. Verlag Heinz Postenrieder, 1975, 28